# EUROPEAN PATENT SPECIFICATION

(11) **EP 3 146 898 B1**
(45) Date of publication and mention of the grant of the patent: **14.11.2018**
(21) Application number: 16190406.5
(22) Date of filing: 23.09.2016
(51) Int. Cl.: A61B 5/145, A61B 5/00, A61B 3/00, A61B 5/15, G02C 7/04

(54) **QUANTUM-DOT SPECTROMETERS FOR USE IN BIOMEDICAL DEVICES AND METHODS OF USE**
QUANTENPUNKT-SPEKTROMETER ZUR VERWENDUNG IN BIOMEDIZINISCHEN VORRICHTUNGEN UND VERFAHREN ZUR VERWENDUNG
SPECTROMETRES A POINTS QUANTIQUES A UTILISER DANS DES DISPOSITIFS BIOMEDICAUX ET PROCEDES D'UTILISATION

(30) Priority: 24.09.2015 US 201514863933
(43) Date of publication of application: 29.03.2017
(73) Proprietor: Johnson & Johnson Vision Care, Inc., Jacksonville, FL 32256 (US)
(72) Inventor: FLITSCH, Frederick A., New Windsor, NY 12553 (US); GONZALEZ, Jorge, Washington D.C, 20017 (US); PUGH, Randall B., Jacksonville, FL 32256 (US)
(74) Representative: Carpmaels & Ransford LLP

(56) References cited:
- EP-A1- 2 843 461
- US-A1- 2014 343 387
- NICHOLAS M. FARANDOS ET AL: "Contact Lens Sensors in Ocular Diagnostics", ADVANCED HEALTHCARE MATERIALS, vol. 4, no. 6, 17 November 2014 (2014-11-17), pages 792-810, XP055342493, DE ISSN: 2192-2640, DOI: 10.1002/adhm.201400504
- JIE BAO ET AL: "A colloidal quantum dot spectrometer", NATURE, vol. 523, no. 7558, 2 July 2015 (2015-07-02), pages 67-70, XP055344555, United Kingdom ISSN: 0028-0836, DOI: 10.1038/nature14576

## Description

### BACKGROUND OF THE INVENTION

### 1. Field of the Invention

Quantum-dot spectrometers for use in biomedical devices are described herein. In some exemplary embodiments, the devices' functionality involves collecting biometric information to perform personalized bioanalysis for the user of the device.

### 2. Discussion of the Related Art

Recently, the number of medical devices and their functionality has begun to rapidly develop. These medical devices may include, for example, implantable pacemakers, electronic pills for monitoring and/or testing a biological function, surgical devices with active components, contact lenses, infusion pumps, and neurostimulators. These devices are often exposed to and interact with biological and chemical systems making the devices optimal tools for collecting, storing, and distributing biometric data.

Some medical devices include components such as semiconductor devices that perform a variety of functions including biometric collection, and may be incorporated into many biocompatible and/or implantable devices. Such semiconductor components require energy and, thus, energization elements must also be included in such biocompatible devices. The addition of self-contained energy in a biomedical device capable of collecting biometric information would enable the device to perform personalized biometric analysis for the user of the device.

EP 2843461 discusses ophthalmic devices with media inserts that have fluorescence based analysis elements upon or within them, which may be utilized to analyze and monitor fluid for certain components. More specifically, the fluorescence based analysis elements may be useful for analyzing an analyte such as glucose in a fluid sample.

US 2014/343387 discusses a system for an energized ophthalmic device with a media insert including microfluidic elements. The microfluidic elements may be useful for the purpose of analyzing an analyte such as glucose in a fluid sample. In addition, some embodiments of the system can function with a medicament administering device to treat an abnormal condition identified during the analyte analysis in the fluid sample.

Jie Bao et al discuss, in "A colloidal quantum dot spectrometer," the performance of a microspectrometer in which interferometric optics are replaced with a two-dimensional absorptive filter array comprised of colloidal quantum dots.

Nicholas M. Farandos et al review, in "Contact Lens Sensors in Ocular Diagnostics," the state-of-the art in contact lens sensor fabrication, their detection, wireless powering, and readout mechanisms, and integration with mobile devices and smartphones.

One aspect of biometric information collection has focused on the ability to pair an analyte to a corresponding enzyme such as glucose to glucose oxidase for the detection of glucose in a fluid medium. Another aspect of biometric information collection focuses on the use of light where a light source shines light through a medium which is in turn collected by a detector and analyzed for the amount of light absorbed, similar to a spectrometer. Spectrometers are widely used in physical, chemical, and biological research; however, current microspectrometer designs mostly use interference filters and interferometric optics that limit their photon efficiency, resolution, and spectral range. Nevertheless, the miniaturization possible with the development of techniques and reagents that utilize quantum-dots in supporting the

acquisition of spectroscopic data may allow for significant advances in the ability of biomedical devices to sense chemical states of their environments.

A quantum-dot (QD) is a nanocrystal commonly made of semiconductor materials. When crystals are "nano-sized" they become small enough to exhibit quantum mechanical properties. Technologies around QDs exploit this quantum mechanical behavior to result in interesting optical properties for the QDs. Therefore, novel devices for biomedical purposes for the use of quantum-dots and for quantum-dot spectrometry may be useful.

### SUMMARY OF THE INVENTION

A biomedical device according to claim 1 is provided. Accordingly, devices and methods for the use of QDs as emission sources, filters, dyes and as narrow and broadband spectrometers on or in powered biomedical devices may enable the powered biomedical devices to specifically and accurately detect analytes on or in the body of a user. Quantum-dots are extremely small entities that can be manufactured with high levels of consistency and purity. Since the quantum-dot manufacturing process may be tuned to different sizes and materials a nearly arbitrary amount of frequencies may be tuned for the spectral response from a type of QD. As emission sources, therefore, fine line fluorescence sources may be formed from the excitation of QDs with their resultant high yield fluorescence emission. For the use of QDs as filters, a tunable transmission response may be obtained. Therefore, it may be easy to create spectrometers comprising hundreds of unique and tuned spectral filters to create the perspective of broadband spectroscopy. Still further spectral relevance of QDs may arise from the fact that individual QDs may have molecules that bind to the surface and quench their fluorescence. These quenching molecules may be selected and designed to bind to analytes and in so doing decouple from the QD that they are quenching, resulting in sensitive fluorescence probes for analyte study.

One general aspect includes forming a biomedical device including an energization element including a first and second current collector, a cathode an anode and an electrolyte. The biomedical device may also comprise a quantum-dot spectrometer which may include a quantum-dot light emitter, a photodetector, and a means of communicating information from the quantum-dot spectrometer to a user. The quantum-dot spectrometer is powered by the energization element. The biomedical device may also include an insert device. This insert creates an encapsulation that
isolates the energization elements from the biomedical environment that this biomedical device operates within.

The biomedical device may further comprise a micro-fluidic pump. The micro-fluidic pump functions to bring a sample of fluid towards or away from the quantum-dot spectrometer when the spectrometer is used for analysis. In some examples the biomedical device is an ophthalmic device. In some examples the biomedical device is a contact lens. In some examples the biomedical device is an electronic pill.

Implementations also include a method of analyzing analytes. The method may include fabricating a quantum-dot light emitter into a biomedical device. As well, a photodetector may be included into a biomedical device. The method may include connecting the quantum-dot emitter and photodetector to an integrated circuit controller where this controller is capable of directing the functionality of the quantum-dot emitter and photodetector. The method includes emitting a narrow wavelength band from the quantum-dot light emitter. The method includes receiving transmitted photons into the photodetector. The method continues with analyzing the absorbance of an analyte based on the intensity of photons received. The biomedical device comprises an energization element which includes a first and second current collector, a cathode, and anode and an electrolyte where the spectrometer is powered by the energization element. The method may further comprise pumping a sample of analytes into the quantum-dot spectrometer cannel before analyzing the analytes. In some implementations, the method may involve the examples where the biomedical device is a contact lens. The method may also involve examples where the biomedical device is an electronic pill.

One general aspect includes a biomedical device comprising an energization element. The biomedical device includes an external encapsulation boundary. The external encapsulation boundary includes a reentrant cavity which creates an external region that is generally surrounded by the biomedical device while allowing fluid to flow in and out from the environment of the biomedical device. The encapsulation layers of the biomedical device allow light to pass through them in important spectral bands. The reentrant channel may be lined by photon emitters and detectors.

The biomedical device includes a quantum-dot light emitter installed to emit light through one side of the sidewall of the cavity through the intervening space of the cavity. The light further proceeds through the opposite or distal side of the sidewall of the cavity. On the other side may be numerous photodetectors installed within the external encapsulation boundary. The biomedical device also includes a radio frequency transceiver and an analog-to-digital converter. A signal from a photodetector is converted by the analog-to-digital converter into a data value that may be transmitted by the radio frequency transceiver. In some examples the biomedical device is a contact lens or an electronic pill. In examples of an electronic pill, the pill may also comprise a release mechanism controllable to release medicament. The detector may form a feedback loop for the device and therefore may adjust the amount of medicament dispersed by the pill.

In some examples of a biomedical device comprising a QD spectrometer, the signal received at the photodetector may be converted to a digital signal and communicated to an external receiver. This external receiver may include a processor that executes an algorithm which calculates a concentration of an analyte and then determines the concomitant release in medicament that is desired. The external receiver transmits data and control signals to the biomedical device.

Implementations include a biomedical device including an energization element. The biomedical device also includes an external encapsulation boundary wherein at least a portion of the boundary comprises an electrically controlled pore. The pore is operative to allow a fluid sample to pass into the biomedical device from an external region. The biomedical device also includes a microfluidic processing chip which mixes fluid samples and reagents. Reagents in the microfluidic processing chip may include analyte specific dyes. The biomedical device includes a quantum-dot light emitter which emits light through a portion of the microfluidic processing chip. The device also includes a photodetector installed on a distal position of the microfluidic processing chip. The device also includes a radio frequency transceiver. The implement may also include an analog to digital converter where a signal from the photodetector may be converted to a digital data value that is transmitted outside the biomedical device by the radio frequency transceiver. These examples include biomedical devices which are contact lenses, electronic pills and electronic pills capable to release medicament based on the signal received at the photodetector.

One general aspect includes a biomedical device as an electronic pill, where the electronic pill includes a release mechanism controllable to release a quantum-dot dye into the cavity, where the dye reacts with analyte molecules and allows the quantum light emitter to excite the quantum-dot dye to emit light. The biomedical device also includes an energization element; an external encapsulation boundary, where at least a portion of the boundary includes an electrically controlled pore operative to allow a fluid sample to pass into the biomedical device from an external region; a microfluidic processing chip operative to mix the fluid sample with a reagent including an analyte specific dye; a quantum-dot light emitter installed to emit light through a portion of the microfluidic processing chip; a photodetector installed on a distal side of the microfluidic processing chip from the quantum-dot light emitter, where light emitted by the quantum-dot light emitter proceeds through a top surface of the microfluidic processing chip, through a sample analysis region of the microfluidic processing chip, through a bottom surface of the microfluidic processing chip and into the photodetector ; a radio frequency transceiver; and an analog-to-digital converter, where a signal from the photodetector is converted to a digital data value that is transmitted. The electronic pill may control its release of medicament. The release of medicament may be adjusted by a controller which acts in response to receipt of converted digital data value.

In some examples the biomedical device comprises a portion that is controllable to release a quantum-dot dye into the microfluidic processing chip. The dye reacts with analyte molecules and the reaction allows the quantum-dot light emitter to excite the quantum-dots without the presence of quenching molecules which might otherwise extinguish the characteristic emission from the quantum-dots.

### BRIEF DESCRIPTION OF THE DRAWINGS

The foregoing and other features and advantages of the invention will be apparent from the following, more particular description of preferred embodiments of the invention, as illustrated in the accompanying drawings:
Figs.1A-1B illustrate exemplary aspects of biocompatible energization elements in concert with the exemplary application of contact lenses.
Fig. 2 illustrates how a spectral band may be analyzed with quantum-dot based filters.
Fig. 3 illustrates a processor that may be used to implement some embodiments of the present invention.
Fig. 4 illustrates an exemplary functional structure model for a biomedical device for a Quantum-Dot Spectrometer.
Fig. 5 illustrates an exemplary Quantum-Dot Spectrometer device.
Fig. 6A illustrates a top view of an exemplary multi-piece annular shaped form insert.
Fig. 6B illustrates a first amplified partial cross sectional representation of the exemplary multi-piece annular shaped form insert of Fig. 6A.
Fig. 6C illustrates a second amplified partial cross sectional representation of the exemplary multi-piece annular shaped form insert of Fig. 6A.
Fig. 7 illustrates a magnified top view partial section of the Quantum-dot Spectrometer System of with an exemplary pumping mechanism as well as sampling regions and controlling components.
Fig. 8 illustrates a top view partial section of an exemplary Quantum-dot Spectrometer System with a fluid sample being flowed through the microfluidic analysis component.
Fig. 9 illustrates a top view section of an exemplary Quantum-dot Spectrometer System component with a waste storage element.
Fig. 10 illustrates a top view section of an exemplary pumping mechanism for a Quantum-dot Spectrometer System using lab on a chip component.
Figs. 11A-C illustrate an exemplary Quantum-Dot Spectrometer in a biomedical device.
Fig. 12 illustrates an exemplary quantum-dot based fluorescence dye.
Fig. 13 illustrates an exemplary flow diagram for sample analyte detection by quantum-dot based spectroscopy.
Fig. 14 illustrates exemplary method steps that may be used to monitor analyte levels of a user wearing the ophthalmic lens according to aspects of the present disclosure.
Fig. 15 illustrates exemplary method steps that may be used to treat the glucose levels of a user wearing the ophthalmic lens according to aspects of the present disclosure.

### DETAILED DESCRIPTION OF THE INVENTION

Spectroscopy utilizing quantum-dots as emission sources, filters and dyes which may be used in biomedical devices are disclosed in this application. In the following sections, detailed descriptions of various examples are described. The descriptions are exemplary embodiments only, and various modifications and alterations may be apparent to those skilled in the art. Therefore, the examples do not limit the scope of this application. Quantum-dot based spectrometers for use in biomedical devices, and the structures that contain them, may be designed for use in devices such as ophthalmic lenses and electronic pills. In some examples, spectroscopy methods utilizing quantum-dots for use in biomedical devices may be designed for use in, or proximate to, the body of a living organism.

### Glossary

In the description and claims below, various terms are used for which the following definitions will apply:
"Anode" as used herein refers to an electrode through which electric current flows into a polarized electrical device. The direction of electric current is typically opposite to the direction of electron flow. In other words, the electrons flow from the anode into, for example, an electrical circuit.

"Binder" as used herein refers to a polymer that is capable of exhibiting elastic responses to mechanical deformations and that is chemically compatible with other energization element components. For example, binders may include electroactive materials, electrolytes, polymers, and the like.

"Biocompatible" as used herein refers to a material or device that performs with an appropriate host response in a specific application. For example, a biocompatible device does not have toxic or injurious effects on biological systems.

"Cathode" as used herein refers to an electrode through which electric current flows out of a polarized electrical device. The direction of electric current is typically opposite to the direction of electron flow. Therefore, the electrons flow into the cathode of the polarized electrical device, and out of, for example, the connected electrical circuit.

"Coating" as used herein refers to a deposit of material in thin forms. In some uses, the term will refer to a thin deposit that substantially covers the surface of a substrate it is formed upon. In other more specialized uses, the term may be used to describe small thin deposits in smaller regions of the surface.

"Electrode" as used herein may refer to an active mass in the energy source. For example, it may include one or both of the anode and cathode.

"Energized" as used herein refers to the state of being able to supply electrical current or to have electrical energy stored within.

"Energy" as used herein refers to the capacity of a physical system to do work. Many uses of the energization elements may relate to the capacity of being able to perform electrical actions.

"Energy Source" or "Energization Element" or "Energization Device" as used herein refers to any device or layer which is capable of supplying energy or placing a logical or electrical device in an energized state. The energization elements may include batteries. The batteries may be formed from alkaline type cell chemistry and may be solid-state batteries or wet cell batteries.

"Fillers" as used herein refer to one or more energization element separators that do not react with either acid or alkaline electrolytes. Generally, fillers may include substantially water insoluble materials such as carbon black; coal dust; graphite; metal oxides and hydroxides such as those of silicon, aluminum, calcium, magnesium, barium, titanium, iron, zinc, and tin; metal carbonates such as those of calcium and magnesium; minerals such as mica, montmorillonite, kaolinite, attapulgite, and talc; synthetic and natural zeolites such as Portland cement; precipitated metal silicates such as calcium silicate; hollow or solid polymer or glass microspheres, flakes and fibers; and the like.

"Functionalized" as used herein refers to making a layer or device able to perform a function including, for example, energization, activation, and/or control.

"Ionizing Salt" as used herein refers to an ionic solid that will dissolve in a solvent to produce dissolved ions in solution. In numerous examples, the solvent may comprise water.

"Mold" as used herein refers to a rigid or semi-rigid object that may be used to form three-dimensional objects from uncured formulations. Some exemplary molds include two mold parts that, when opposed to one another, define the structure of a three-dimensional object.

"Power" as used herein refers to work done or energy transferred per unit of time.

"Rechargeable" or "Re-energizable" as used herein refer to a capability of being restored to a state with higher capacity to do work. Many uses may relate to the capability of being restored with the ability to flow electrical current at a certain rate for certain, reestablished time periods.

"Reenergize" or "Recharge" as used herein refer to restoring to a state with higher capacity to do work. Many uses may relate to restoring a device to the capability to flow electrical current at a certain rate for a certain reestablished time period.

"Released" as used herein and sometimes referred to as "released from a mold" means that a three-dimensional object is either completely separated from the mold, or is only loosely attached to the mold, so that it may be removed with mild agitation.

"Stacked" as used herein means to place at least two component layers in proximity to each other such that at least a portion of one surface of one of the layers contacts a first surface of a second layer. In some examples, a coating, whether for adhesion or other functions, may reside between the two layers that are in contact with each other through said coating.

"Traces" as used herein refer to energization element components capable of connecting together the circuit components. For example, circuit traces may include copper or gold when the substrate is a printed circuit board and may typically be copper, gold or printed film in a flexible circuit. A special type of "Trace" is the current collector. Current collectors are traces with electrochemical compatibility that make the current collectors suitable for use in conducting electrons to and from an anode or cathode in the presence of electrolyte.

The methods and apparatus presented herein relate to forming biocompatible energization elements for inclusion within or on flat or three-dimensional biocompatible devices. A particular class of energization elements may be batteries that are fabricated in layers. The layers may also be classified as laminate layers. A battery formed in this manner may be classified as a laminar battery.

There may be other examples of how to assemble and configure batteries according to the present invention, and some may be described in following sections. However, for many of these examples, there are selected parameters and characteristics of the batteries that may be described in their own right. In the following sections, some characteristics and parameters will be focused upon.

Recent developments in biomedical devices including, for example, ophthalmic lenses, have enabled functionalized biomedical devices that may be energized. The energized biomedical devices may comprise the necessary elements to collect and analyze analytes of users using embedded micro-electronics. Additional functionality using micro-electronics may include, for example, audio, visual, and haptic feedback to the user. In some embodiments, the quantum-dot spectrometers for use in biomedical devices may be in wireless communication with one or more wireless device(s) and receive signal data that may be used in real time for the determination of an abnormal analyte concentration and correlated cause. The wireless device(s) may include, for example, a smart phone device, a tablet, a personal computer, a FOB, an MP3 player, a PDA, and other similar devices.

### Energized Ophthalmic Device

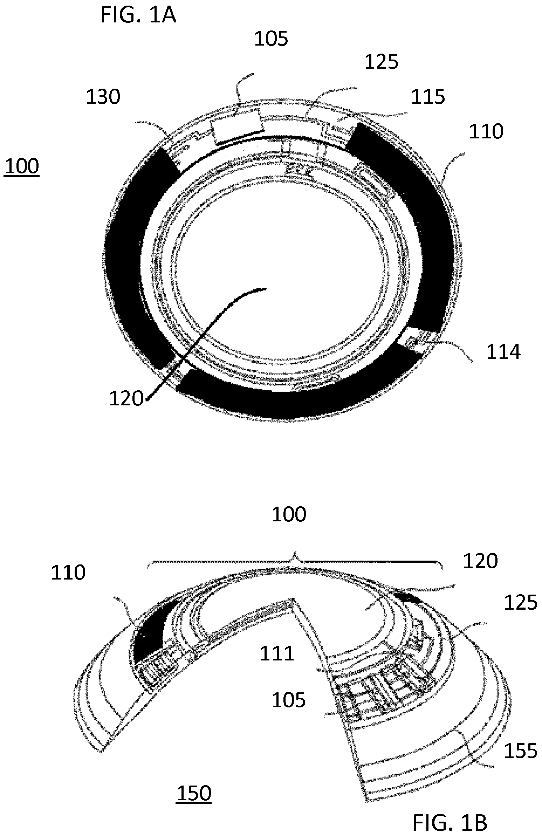

Referring to Fig. 1A, an exemplary embodiment of a media insert 100 for an energized ophthalmic device and a corresponding energized ophthalmic device 150 (Fig. 1B) are illustrated to provide an example of an energized biomedical device structure that may support the operation of quantum-dot based spectroscopy. The media insert 100 may comprise an optical zone 120 that may or may not be functional to provide vision correction. Where the energized function of the ophthalmic device is unrelated to vision, the optical zone 120 of the media insert may be void of material. In some exemplary embodiments, the media insert include a portion not in the optical zone 120 comprising a substrate 115 incorporated with energization elements 110 (power source) and electronic components 105, such as a spectrometer. The energization elements 110 may be connected to a circuit element that have its own substrate 111 upon which interconnect features 125 be located. The circuit, which may be in the form of an integrated circuit, be electrically and physically connected to the substrate 111 and its interconnect features 125. The energization elements 110 have their own interconnect features to join together elements as be depicted underlying the region of interconnect 114.

In some exemplary embodiments, a power source or energization elements 110 (for example a battery) and a load such as electronic components 105 (for example a spectrometer) are attached to the substrate 115. Conductive traces called interconnect features 125 and 130 electrically interconnect the electronic components 105 and the energization elements 110. The media insert 100 be fully encapsulated to protect and contain the energization elements 110, interconnect features 125, and electronic components 105, such as a spectrometer. In some exemplary embodiments, the encapsulating material is semi-permeable, for example, to prevent specific substances, such as water, from entering the media insert and to allow specific substances, such as ambient gasses or the byproducts of reactions within energization elements, to penetrate or escape from the media insert.

In some exemplary embodiments, as depicted in Fig. 1B, the media insert 100 be included in an ophthalmic device 150, which comprise a polymeric biocompatible material. The ophthalmic device 150 includes a rigid center, soft skirt design wherein the central rigid optical element comprises the media insert 100. In some specific embodiments, the media insert 100 be in direct contact with the atmosphere and the corneal surface on respective anterior and posterior surfaces, or alternatively, the media insert 100 may be encapsulated in the ophthalmic device 150. The periphery 155 of the ophthalmic device 150 or lens may be a soft skirt material, including, for example, a hydrogel material. The infrastructure of the media insert 100 and the ophthalmic device 150 provide an environment for numerous embodiments involving fluid sample processing with quantum-dot based analysis elements while isolating the internal components from the biomedical environment surrounding the insert.

### Fluorescence Based Probe Elements for Analyte Analysis

Various types of analytes may be detected and analyzed using fluorescence based analysis techniques. A subset of these techniques involves the direct fluorescence emission from the analyte itself. A more generic set of techniques relate to fluorescence probes that have constituents that bind to analyte molecules, and in so binding, alter a fluorescence signature. For example, in Forster Resonance Energy Transfer (FRET), probes are configured with a combination of two fluorophores that are chemically attached to interacting proteins. The distance of the fluorophores from each other can affect the efficiency of a fluorescence signal emanating therefrom.

One of the fluorophores absorbs an excitation irradiation signal and can resonantly transfer the excitation to electronic states in the other fluorophore. The binding of analytes to the attached interacting proteins may disturb the geometry and cause a change in the fluorescent emission from the pair of fluorophores. Binding sites may be genetically programmed into the interacting proteins, and for example, a binding site, which is sensitive to glucose, may be programmed. In some cases, the resulting site may be less sensitive or non-sensitive to other constituents in interstitial fluids of a desired sample.

The binding of an analyte to the FRET probes may yield a fluorescence signal that is sensitive to glucose concentrations. In some exemplary embodiments, the FRET based probes be sensitive to as little as a 10 uM concentration of glucose and be sensitive to concentrations of up to hundreds of micromolar. Various FRET probes may be genetically designed and formed. The resulting probes are configured into structures that assist analysis of interstitial fluids of a user. In some exemplary embodiments, the probes are placed within a matrix of material that is permeable to the interstitial fluids and their components, for example, the FRET probes may be assembled into hydrogel structures. In some exemplary embodiments, these hydrogel probes are included into the hydrogel based processing of ophthalmic contact lenses in such a manner that they reside in a hydrogel encapsulation that is immersed in tear fluid when worn upon the eye. In other exemplary embodiments, the probe is inserted in the ocular tissues just above the sclera. A hydrogel matrix comprising fluorescence emitting analyte sensitive probes may be placed in various locations that are in contact with bodily fluids containing an analyte.

In the examples provided, the fluorescence probes are in contact with interstitial fluid of the ocular region near the sclera. In these cases, where the probes are invasively embedded, a sensing device provide a radiation signal incident upon the fluorescence probe from a location external to the eye such as from an ophthalmic lens or a hand held device held in proximity to the eye.

In other exemplary embodiments, the probe is embedded within an ophthalmic lens in proximity to a fluorescence-sensing device that is also embedded within the ophthalmic lens. In some exemplary embodiments, a hydrogel skirt encapsulates both an ophthalmic insert with a fluorescence detector as well as a FRET based analyte probe.

### Quantum-Dot Spectroscopy

Small spectroscopy devices may be of significant aid in creating biomedical devices with the capability of measuring and controlling concentrations of various analytes for a user. For example, the metrology of glucose may be used to control variations of the material in patients and after treatments with medicines of various kinds. Current microspectrometer designs mostly use interference filters and interferometric optics to measure spectral responses of mixtures that contain materials that absorb light. In some examples a spectrometer is formed by creating an array composed of quantum-dots. A spectrometer based on quantum-dot arrays may measure a light spectrum based on the wavelength multiplexing principle. The wavelength multiplexing principle is accomplished when multiple spectral bands are encoded and detected simultaneously with one filter element and one detector element, respectively. The array format allows the process to be efficiently repeated many times using different filters with different encoding so that sufficient information is obtained to enable computational reconstruction of the target spectrum. An example may be illustrated by considering an array of light detectors such as that found in a CCD camera. The array of light sensitive devices is useful to quantify the amount of light reaching each particular detector element in the CCD array. In a broadband spectrometer, a plurality, sometimes hundreds, of quantum-dot based filter elements are deployed such that each filter allows light to pass from certain spectral regions to one or a few CCD elements. An array of hundreds of such filters laid out such that an illumination light passed through a sample proceed through the array of QD Filters and on to a respective set of CCD elements for the QD filters. The simultaneous collection of spectrally encoded data allow for a rapid analysis of a sample.

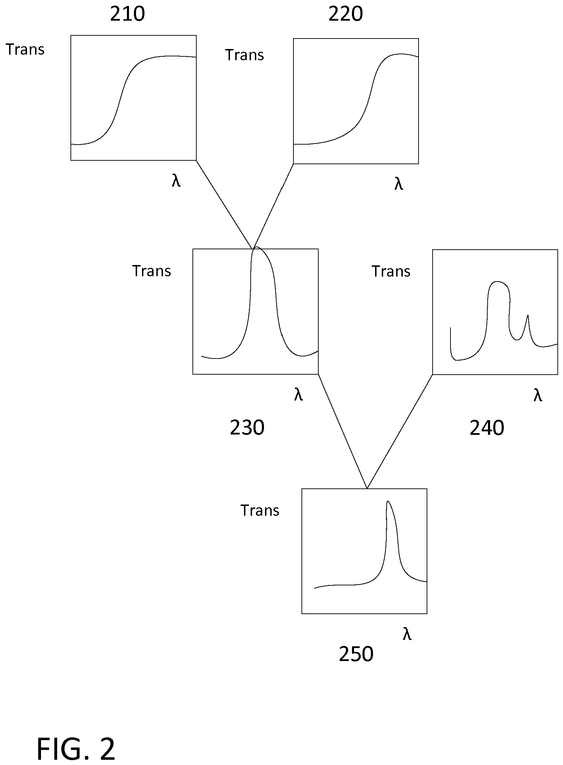

Narrow band spectral analysis examples may be formed by using a smaller number of QD filters surrounding a narrow band. In Fig. 2 an illustration of how a spectral band may be observed by a combination of two filters is illustrated. It also is clear that the array of hundreds of filters may be envisioned as a similar concept to that in Fig. 2 repeated many times.

If Fig. 2, a first QD filter 210 have an associated spectral transmission response as illustrated and indicated as Trans. A second QD filter 220 have a shifted associated spectral transmission associated with a different nature of the quantum-dots included in the filter, for example the QDs may have a larger diameter in the QD filter of 220. The difference curve of a flat irradiance of light of all wavelength (white light) result from the difference of the absorption result from light that traverses filter 220 and that traverses filter 210. Thus, the effect of irradiating through these two filters is that the difference curve would indicate spectral response in the band 230 depicted. When an analyte is introduced into the light path of the spectrometer, where the analyte has an absorption band in the UV/Visible spectrum, and possible in the infrared, the result would be to modify the transmission of light in that spectral band as shown by spectrum 240. The difference from 230 to 240 results in a transmission spectrum 250 for the analyte in the region defined by the two quantum-dot filters. Therefore, a narrow spectral response is obtained by a small number of filters. In some examples, redundant coverage by different filter types of the same spectral region may be employed to improve the signal to noise characteristics of the spectral result.

The absorption filters based on QDs may include QDs that have quenching molecules on their surfaces. These molecules stop the QD from emitting light after it absorbs energy in appropriate frequency ranges. More generally, the QD filters may be formed from nanocrystals with radii smaller than the bulk exciton Bohr radius, which leads to quantum confinement of electronic charges. The size of the crystal is related to the constrained energy states of the nanocrystal and generally decreasing the crystal size has the effect of a stronger confinement. This stronger confinement affects the electronic states in the quantum-dot and results in an increased the effective bandgap, which results in shifting to the blue wavelengths both of both optical absorption and fluorescent emission. There have been many spectral limited sources defined for a wide array of quantum-dots that be available for purchase or fabrication and may be incorporated into biomedical devices to act as filters. By deploying slightly modified QDs such as by changing the QD's size, shape and composition it is possible to tune absorption spectra continuously and finely over wavelengths ranging from deep ultraviolet to mid-infrared. QDs can also be printed into very fine patterns.

### Diagrams for Electrical and Computing System

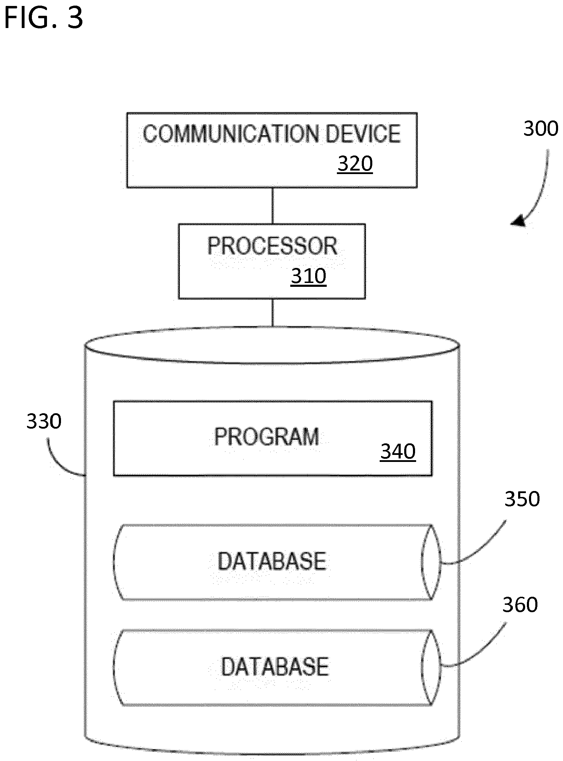

Referring now to Fig. 3, a schematic diagram of a processor that may be used to implement some aspects of the present disclosure is illustrated. The controller 300 includes one or more processors 310, which include one or more processor components coupled to a communication device 320. In some embodiments, a controller 300 is used to transmit energy to the energy source placed in the device.

The processors 310 be coupled to a communication device 320 configured to communicate energy via a communication channel. The communication device 320 is used to electronically communicate with components within the media insert, for example. The communication device 320 may also be used to communicate, for example, with one or more controller apparatus or programming/interface device components.

The processor 310 is also in communication with a storage device 330. The storage device 330 comprise any appropriate information storage device, including combinations of magnetic storage devices, optical storage devices, and/or semiconductor memory devices such as Random Access Memory (RAM) devices and Read Only Memory (ROM) devices.

The storage device 330 stores a program 340 for controlling the processor 310. The processor 310 performs instructions of a software program 340, and thereby operates in accordance with the present invention. For example, the processor 310 receives information descriptive of media insert placement, active target zones of the device. The storage device 330 also stores other pre-determined biometric related data in one or more databases 350 and 360. The database include, for example, predetermined retinal zones exhibiting changes according to cardiac rhythm or an abnormal condition correlated with the retinal vascularization, standard measurement thresholds, metrology data, and specific control sequences for the system, flow of energy to and from a media insert, communication protocols, and the like. The database may also include parameters and controlling algorithms for the control of the biometric based monitoring system that reside in the device as well as data and/or feedback that can result from their action. In some embodiments, that data be ultimately communicated to/from an external reception wireless device.

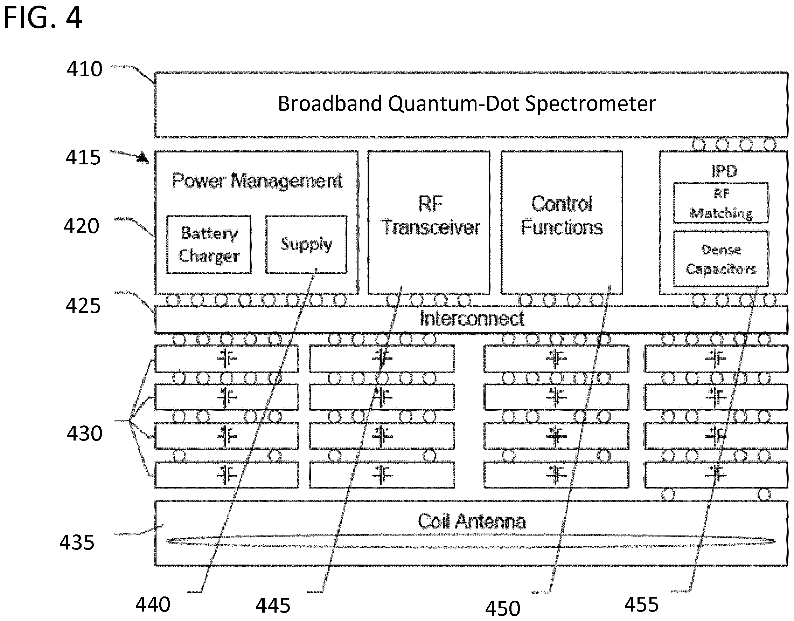

In some embodiments according to aspects of the disclosure, a single and/or multiple discrete electronic devices are included as discrete chips. In other embodiments, energized electronic elements are included in a media insert in the form of stacked integrated components. Referring now to Fig. 4, a schematic diagram of an exemplary cross section of a stacked die integrated components implementing a quantum-dot spectrometer system 410 is depicted. The quantum-dot spectrometer may be, for example, a glucose monitor, a retinal vascularization monitor, a visual scanning monitor, or any other type of system useful for providing spectrophometric information about the user. In particular, a media insert may include numerous layers of different types which are encapsulated into contours consistent with the environment that they will occupy. In some embodiments, these media inserts with stacked integrated component layers assume the entire shape of the media insert. Alternatively in some cases, the media insert occupy just a portion of the volume within the entire shape.

As shown in Fig. 4, there may be thin film batteries 430 used to provide energization. In some embodiments, these thin film batteries 430 comprise one or more of the layers that be stacked upon each other with multiple components in the layers and interconnections there between. The batteries 430 are depicted as thin film batteries for exemplary purposes, there may be numerous other energization elements consistent with the embodiments herein including operation in both stacked and non-stacked embodiments. As a non-limiting alternative example cavity based laminate form batteries with multiple cavities may perform equivalently or similarly to the depicted thin film batteries.

In some embodiments, there be additional interconnections between two layers that are stacked upon each other. In the state of the art there be numerous manners to make these interconnections; however, as demonstrated the interconnection may be made through solder ball interconnections between the layers. In some embodiments only these connections are required; however, in other cases the solder balls contact other interconnection elements, as for example with a component having through layer vias.

In other layers of the stacked integrated component media insert, a layer 425 may be dedicated for the interconnections of two or more of the various components in the interconnect layers. The interconnect layer 425 contain, vias and routing lines that can pass signals from various components to others. For example, interconnect layer 425 provide the various battery elements connections to a power management unit 420 that may be present in a technology layer 415. The power management unit 420 has circuitry dedicated to supplying voltage sources with controlled characteristics 440. Other components in the technology layer 415 include, for example, a transceiver 445, control components 450 and the like. In addition, the interconnect layer 425 may function to make connections between components in the technology layer 415 as well as components outside the technology layer 415; as may exist for example in the integrated passive device 455. There be numerous manners for routing of electrical signals that be supported by the presence of dedicated interconnect layers such as interconnect layer 425.

In some embodiments, the technology layer 415, like other layer components, is included as multiple layers as these features represent a diversity of technology options that may be included in media inserts. In some embodiments, one of the layers includes CMOS, BiCMOS, Bipolar, or memory based technologies whereas the other layer includes a different technology. Alternatively, the two layers may represent different technology families within a same overall family; as for example one layer include electronic elements produced using a 0.5 micron CMOS technology and another layer include elements produced using a 20 nanometer CMOS technology. It is apparent that many other combinations of various electronic technology types would be consistent within the art described herein.

In some embodiments, the media insert include locations for electrical interconnections to components outside the insert. In other examples, however, the media insert also include an interconnection to external components in a wireless manner. In such cases, the use of antennas in an antenna layer 435 provides exemplary manners of wireless communication. In many cases, such an antenna layer 435 is located, for example, on the top or bottom of the stacked integrated component device within the media insert.

In some of the embodiments discussed herein, the energization elements such as batteries 430 may be included as elements in at least one of the stacked layers themselves. It be noted as well that other embodiments are possible where the battery elements 430 are located externally to the stacked integrated component layers. Still further diversity in embodiments derive from the fact that a separate battery or other energization component may also exist within the media insert, or alternatively these separate energization components may also be located externally to the media insert. In these examples, the functionality be depicted for inclusion of stacked integrated components, it be clear that the functional elements may also be incorporated into biomedical devices in such a manner that does not involve stacked components and still be able to perform functions related to the embodiments herein.

Components of the quantum-dot spectrometer system 410 may also be included in a stacked integrated component architecture. In some embodiments, the quantum-dot spectrometer system 410 components are attached as a portion of a layer. In other embodiments, the entire quantum-dot spectrometer system 410 also comprises a similarly shaped component as the other stacked integrated components. In some alternative examples, the components not be stacked but laid out in the peripheral regions of the ophthalmic device or other biomedical device, where the general functional interplay of the components function equivalently however the routing of signals and power through the entire circuit differ.

When constructing a quantum-dot spectrometer system 410 in a biomedical device, size may be an integral factor. Quantum-dot emitters may be fashioned in a manner similar to the formation of light emitting diodes. Layers of materials surround the quantum-dots to create light emitting diodes with the quantum-dots. Organic layers act as electron donors and as hole donors into the quantum-dot layer. In a non-limiting example, the QDs be sandwiched between electron transport layers and hole transport layers. Application of electric potential to electrodes connected to the electron transport layer and the hold transport layer excite the QD into photoluminescence at a wavelength band characteristic of the QDs. Examples of electron transport layers and hole transport layers include tris-(8-hydroxyquinoline) aluminum; bathocuproine; 4,4'-N,N'-dicarbazolylbiphenyl; poly(2-(6-cyano-6'-methylheptyloxy)- 1,4-phenylene); poly[(9,9-dihexylfluorenyl-2,7-diyl)-co-(1,4-{benzo-[2,1',3]thiadiazole})]; poly[2-methoxy-5-(2-ethylhexyloxy)-1,4-phenylenevinylene]; 4,4-bis[N-(1-naphyl)-N-phenylamino] biphenyl; 2-(4-biphenylyl)-5-(4-tertbutylphenyl)-1,3,4 oxadiazole; poly∼3,4-ethylene dioxythiophene; poly(9,9'-dioctylfluorene-co-N-(4-butylphenyl)diphenylamine; perfluoro-cyclobutane; poly(phenylene vinylene); 3-(4-Biphenylyl)-4-phenyl-5-tertbutylphenyl- 1, 2, 4-triazole; Poly[(9,9-dioctylfluorenyl-2,70diyl)-co-(4-4'-(N-(4-secbutylphenyl)) diphenylamine)]; 1,3,5-tris(N-phenylbenzimidazole-2-yl)-benzene; and N, N'-diphenyl-N, N'-bis(3-methylphenyl)-(1,1'-biphenyl)-4,4'-diamine as non-limiting examples.

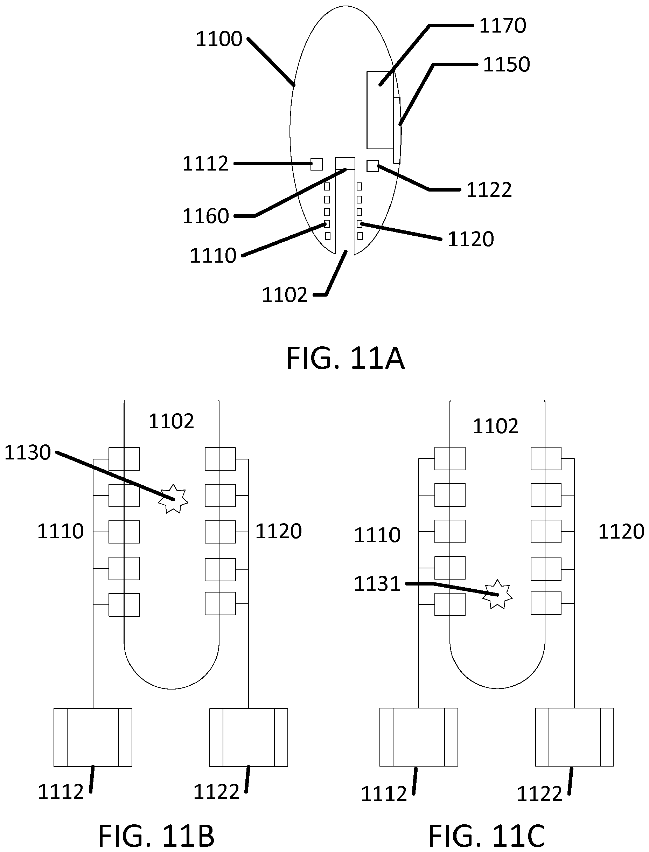

Once excited by a current (such as from the power management unit 420), the quantum-dot layer emit light at a designed specified wavelength from the quantum-dot spectrometer system 410. The emitted light interacts with an outside environment, or with a specific sample or samples in the environment, wherein the sample or samples absorb the emitted light at certain wavelengths. The quantum-dot spectrometer then receive the remaining light, which has been transmitted through the sample or samples, in a quantum-dot detector (refer to Fig. 11A for one exemplary embodiment) within the quantum-dot spectrometer system 410.

Similarly, millimeter or nanometer sized quantum-dot detectors may be implemented into a quantum-dot spectrometer system 410 (see also, Fig. 11A, at 1120). Current quantum-dot detectors rely on charged-coupled devices (CCD); however, CCDs do not currently provide the size scale required for millimeter- or nanometer sized quantum-dot spectrometers. Rather, smaller photodiode arrays may be used to achieve the size requirements. Photodiodes are semiconductor devices that convert light into energy. Millimeter or nanometer sized photodiodes may be constructed through photolithographic means.

### Biomarkers/Analytical Chemistry

A biomarker, or biological marker, generally refers to a measurable indicator of some biological state or condition. The term is also occasionally used to refer to a substance the presence of which indicates the existence of a living organism. Further, life forms are known to shed unique chemicals, including DNA, into the environment as evidence of their presence in a particular location. Biomarkers are often measured and evaluated to examine normal biological processes pathogenic processes, or pharmacologic responses to a therapeutic intervention. In their totality, these biomarkers reveal vast amounts of information important to the prevention and treatment of disease and the maintenance of health and wellness.

Biomedical devices configured to analyze biomarkers be utilized to quickly and accurately reveal one's normal body functioning and assess whether that person is maintaining a healthy lifestyle or whether a change be required to avoid illness or disease. Biomedical devices may be configured to read and analyze proteins, bacteria, viruses, changes in temperature, changes in pH, metabolites, electrolytes, and other such analytes used in diagnostic medicine and analytical chemistry.

### Ophthalmic Insert Devices and Other Biomedical Devices with Quantum-Dot Spectrometer

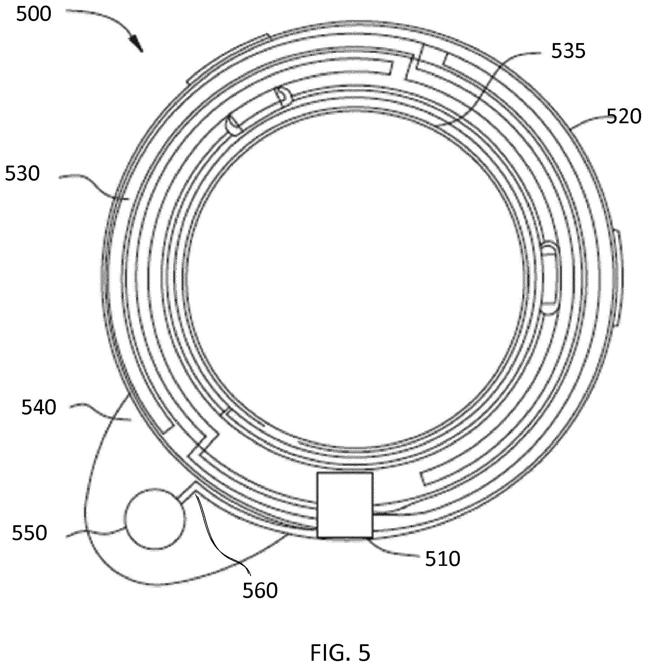

Referring to Fig. 5, an ophthalmic insert 500 is demonstrated including components that form an exemplary quantum-dot (QD) spectrometer system. The demonstrated ophthalmic insert is shown in an exemplary annular form having an internal border of 535 and an external border of 520. In addition to energization elements such as the battery power source 530, control circuitry 510, and interconnect features 560 there be a broadband QD spectrometer system 550, which in certain exemplary embodiments be positioned on a flap 540. The flap 540 be connected to the insert 500 or be an integral, monolithic extension thereof. The flap 540 properly positions the broadband QD spectrometer system 550 when an ophthalmic device comprising a QD detector is worn. The flap 540 allows the broadband QD spectrometer system 550 to overlap with portions of the user's eye away from the optic zone. The broadband QD spectrometer system 550 is capable of determining an analyte, in terms of its presence or its concentration, in a fluid sample.

For a broadband QD spectrometer, an analyte sample be exposed to an excitation light source which be passed through an array of different QD filters. This light source with the array of filters is located within the body of the analytical system. In some exemplary embodiments, the light source may comprise a solid-state device or devices similar to a light emitting diode (LED). The QD filtered light source be irradiated through a sample and reflect back on some tissue layers to a detector array

An electronic control bus of interconnect features 560 provide the signals to the light source or sources and return signals from the detectors. The powered electronic component provides the signals and power aspects. The exemplary embodiment of Fig. 5, illustrates a battery power source 530 to the electronic circuitry 510. In other exemplary embodiments, energization also is provided to the electronic circuitry by the coupling of energy through wireless manners such as radiofrequency transfer or photoelectric transfer.

### Methods of Detecting - Microfluidic System

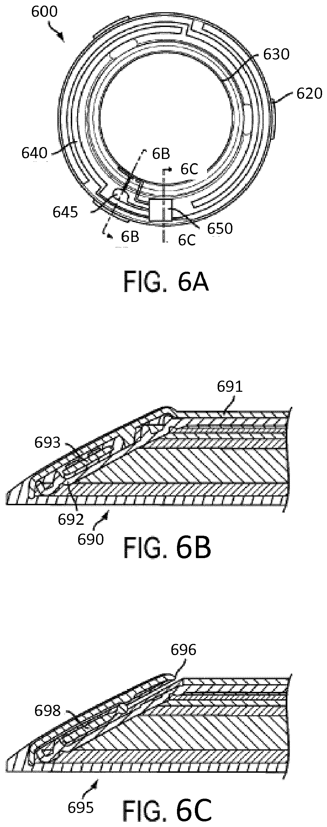

A Microfluidic System may be utilized to pull sample from an outside environment into the biomedical device in order to analyze the sample in a more controlled fashion within an internal region defined by the insert pieces. Referring now to Fig. 6A, a top view of an exemplary multi-piece annular shaped insert 600 is depicted. As depicted, the exemplary multi-piece annular shaped insert 600 be a ring of material around a central optical zone that is devoid of material. Moreover, the annular shaped insert 600 be defined by an exterior extent 620 and an internal annulus edge 630. Included in between the exterior extend 620 and the internal annulus edge 630 are found energization elements 640, interconnect features 645 of various types and/or an electronic circuit element 650.

Referring now to Fig. 6B, a first amplified partial cross sectional representation 690 of the exemplary multipiece annular shaped form insert 600 of Fig. 6A is depicted. The cross section 690 reveals that the annular shaped insert 600 as a combination of a front insert piece 691 and a rear insert piece 692. As depicted, in some embodiments, the front insert piece 691 and the rear insert piece 692 be joined and sealed together. In different embodiments, other structural features and means can be implemented to join both pieces together. Also shown in an encapsulated location is an integrated circuit element 693 connected to interconnection elements.

Referring now to Fig. 6C, a second amplified partial cross sectional representation 695 of the exemplary multipiece annular shaped form insert 600 of Fig. 6A is depicted. In particular, in other sections/embodiments, a different type of structure may be found, as depicted in cross section 695. As shown, it may be observed that there is a gap or pore 696 that is formed to allow some portion of the interior of the annular shaped insert 600 to be open to an external environment. There may be numerous components 698 that connect to this opening, and can themselves be encapsulated within the annular shaped insert 600. Accordingly, this ability to allow component(s) 698 situated within the annular shaped insert 600 to controllably interface with fluids and/or gasses in their exterior environment can, in some embodiments, enable for the incorporation of QD spectrometer elements within ophthalmic device.

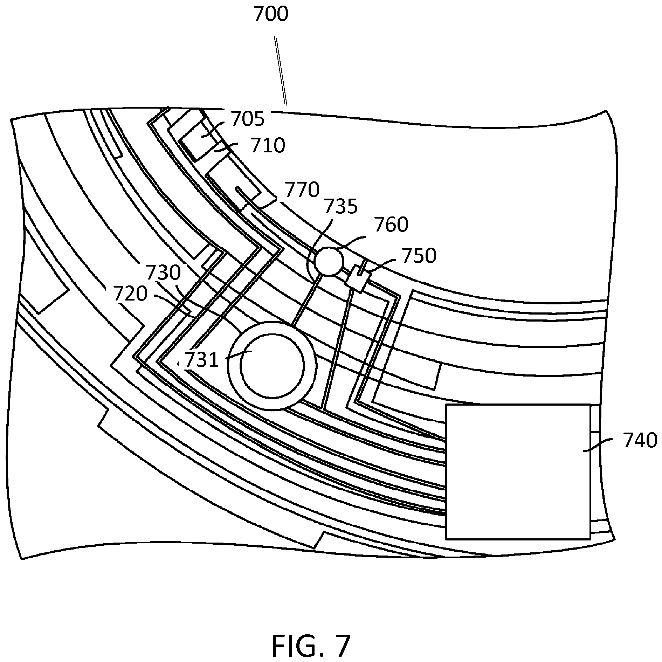

Referring now to Fig. 7, a top view of a section of a Microfluidic Analytical System 700 is illustrated with an exemplary pumping mechanism 760 as well as sampling regions and controlling components is depicted. As shown, in some embodiments control circuitry 740 be electrically connected to components of the micro fluidic analytical system through interconnect(s) 720. A control element 750 for a pore (not shown) be included and be useful for connecting the Microfluidic Analytical System 700 to fluid (not shown) outside of the insert. Exemplary aspects of different designs of pores may be found in following sections; however, the pore allow fluid samples to be passed from outside the insert environment to a pumping element 760.

In some embodiments, the pumping element 760 have an activating or driving component 730 that be capable of engaging the pump element 760. In one example, the pump element 760 comprise a flexible and collapsible membrane that be activated by the application of pressure upon the membrane. There are numerous manners for driving the application of pressure upon the membrane. For example, a fluid may fill a cavity 731 and flow through a tube 735 connecting the cavity 731 to the pumping element 760. Accordingly, the cavity 731 includes features allowing the application of pressure upon the fluid contained within. For example, piezoelectric components may be used to expand volume on the application of voltage thus pressurizing the contained fluid. In other embodiments, thermo-compressive materials respond to a temperature change that be controlled by the application of electric energy to a heating element. In a yet another embodiment, an Electrowetting on Dielectric (EWOD) component exert a pressure on the fluid by a change in the wetting characteristics of a surface in cavity 731 upon the application of a potential. There may also be other means of driving a pump mechanism that also be directly engaged at the pump element 760 itself. Still further diversity may derive from the use of EWOD components to influence the flow of fluids themselves rather than the use of mechanical pumping means.

The pump element 760 forces fluid to flow through a channel 770 and subsequently into an analyzing chamber 705 of the Microfluidic Analytical System 700. Further detail of the components in such chambers 705 will be described in following sections, but briefly stated the fluid flow through the analyzing chamber 705 and cause influences to occur on electrode(s) 710 which may be part of the components.

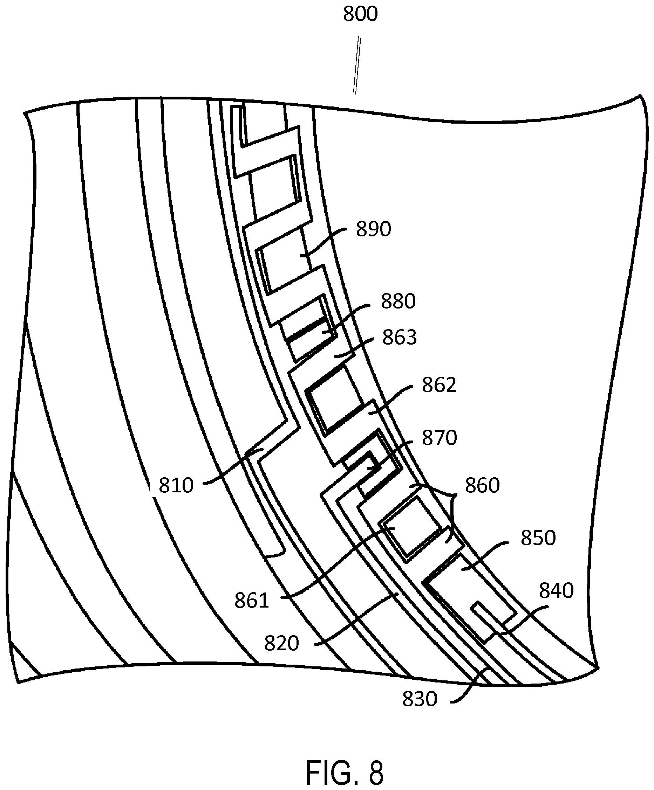

Referring now to Fig. 8, a top view partial section of an exemplary Microfluidic Analytical System 800 with a fluid sample being flowed through the microfluidic analysis component is illustrated. Because of the nature of an annular system, the components may be observed to be deployed in a curvilinear fashion as there may be numerous details that change in a curvilinear system including, for example, the exact shapes of electrodes and chamber cross sections. In other embodiments, however, linear analytical systems be formed that have dimensions that allow them to fit in the ocular environment. Further, in additional embodiments, regardless of the nature of the system along the analysis chamber, the entire substrate that the chamber rests upon can be curved allowing it to rest upon the roughly spherical surface of an eye. The details of the three dimensional nature of the analysis chamber factor into models related to the performance of the systems. For illustration purposes, however, this description declares these nuances, but will illustrate an exemplary embodiment by curving the features of a linear Micro fluidic Analytical System 800. Depicted in the portion of the Micro fluidic Analytical System 800, a micro-channel 850 for receiving and transporting fluid samples is shown. These fluid samples may be pumped, for example, by the previously discussed pumping system (e.g. 760 in Fig. 7) from an external location. For example, fluid samples may be sampled from ocular fluid that surrounds a contact lens containing the Microfluidic Analytic System 800. An analyte sensor 870 is found for example along the micro-channel. This analyte sensor 870 is capable of performing one or more of: an electrochemical analysis step, a photometric analysis step or other analytical steps upon fluid samples. In some examples QD spectrometry is performed in these regions. In an exemplary embodiment, the analysis step relate to a photometric sensing of glucose concentration based on a fluorescence sensor typology using one or more components. In another example, the sensor detects the presence of reaction products from a glucose oxidase interaction with portions of the analyte sensor 870 and the fluid sample. There may be numerous electrical interconnections 820 which connect the sensing element 870 to control electronics.

Fluid flow into the micro-channel 850 from a pump channel 840. As the fluid flows into the micro-channel 850 it displace other fluid in a particular region, or on an initial use displace ambient gas in the channel. As a fluid flows, it is sensed by a pre-sensor micro-channel portion comprising electrodes 860 and 861 as well as a post-sensor portion comprising electrodes 862 and 863. In some embodiments the measurement of impedance between electrodes such as 860 and 861 is used to sense the flow of material. In other embodiments, the resistance of a chain of electrodes 862 and 863 is altered by the presence of a fluid within the micro-channel 850, or the presence of a front between two fluids of different characteristics residing in the micro-channel 850. A fluid 880 flows through the micro-channel from an empty region of the micro-channel 890 to be sampled. Alternatively, micro-channel portion at 890 represent a different solution of fluid that for example have different concentration of electrolytes, and therefore, conductivity than that of typical tear fluid.

In general, measuring impedances, or ohmic resistances, between position electrodes 860-863 in embodiments of the present invention may be accomplished by applying a voltage therebetween and measuring the resulting current. Either a constant voltage or an alternating voltage be applied between the position electrodes 860-863 and the resulting direct current (DC) or alternating current (AC), respectively, measured. The resulting DC or AC current is then used to calculate the impedance or ohmic resistance. Furthermore, one skilled in the art will recognize that measuring impedance may involve measuring both an ohmic drop (i.e., resistance [R] in Ohms or voltage/current) and measuring capacitance (i.e., capacitance in Farads or coulombs/volt). In practice, impedance may be measured, for example, by applying an alternating current to the position electrode(s) 860-863 and measuring the resulting current. At different frequencies of alternating current, either resistive or capacitive effects prevail in determining the measured impedance. The pure resistive component can prevail at lower frequencies while the pure capacitive component can prevail at higher frequencies. To distinguish between the resistive and capacitive components, the phase difference between the applied alternating current and the measured resulting current can be determined. If there is zero phase shift, the pure resistive component is prevailing. If the phase shift indicates that the current lags the voltage, then the capacitive component is significant. Therefore, depending on the frequency of an applied alternating current and position electrode configuration, it can be beneficial to measure either resistance or a combination of resistance and capacitance.

Referring back to the specific example of Fig. 8, impedance measurements be performed by, for example, applying an alternating voltage between first position electrode 830 and a final position electrode connection 810 and measuring the resulting alternating current. Since the chain of electrodes including 860, 861, 862 and 863 can be a portion of a capacitor, (along with any substance [e.g., air or a liquid sample] within micro-channel 850 between subsequent position electrodes and any layers that be separating the position electrodes from direct contact with the fluid in the micro-channel 850), the measured current be used to calculate the impedance. The presence or absence of a liquid sample in micro-channel 850, 890 between electrodes will affect the measured current and impedance. The frequency and amplitude of the alternating voltage applied between a first and second position electrodes 860-863 can be predetermined such that the presence of a liquid sample between a first and second position electrodes 860-863 is detected by a significant increase in measured current.

With respect to the measurement of impedance or resistance, the magnitude of the applied voltage can be, for example, in range from about 10m V to about 2 volts for the circumstance of an ophthalmic tear fluid sample and carbon based or silver-based ink position electrodes. The lower and upper limits of the applied voltage range are dependent on the onset of electrolysis or electrochemical decomposition of the liquid sample. In instances where an alternating voltage is employed, the alternating voltage can be applied, for example, at a frequency that results in a negligible net change in the liquid sample's properties due to one or more electrochemical reaction. Such a frequency range can be, for example, from about 10 Hz to about 100 kHz with a voltage waveform symmetrical around 0 Volts (i.e., the RMS value of the alternating voltage is approximately zero).

As depicted, analyte sensor 870 and position electrodes 860-863 can each be in operative communication with the micro-channel 850. It should be noted that position electrodes 860-863 employed in embodiments of the present invention can be formed of any suitable conductive material known to those skilled in the art, including conductive materials conventionally used as analytical electrode materials and, in particular, conductive materials known as suitable for use in flexible circuits, photolithographic manufacturing techniques, screen printing techniques and flexo-printing techniques. Suitable conductive materials include, for example, carbon, noble metals (e.g., gold, platinum and palladium), noble metal alloys, conductive potential-forming metal oxides and metal salts. Position electrodes can be formed, for example, from conductive silver ink, such as the commercially available conductive silver ink Electrodag 418 SS.

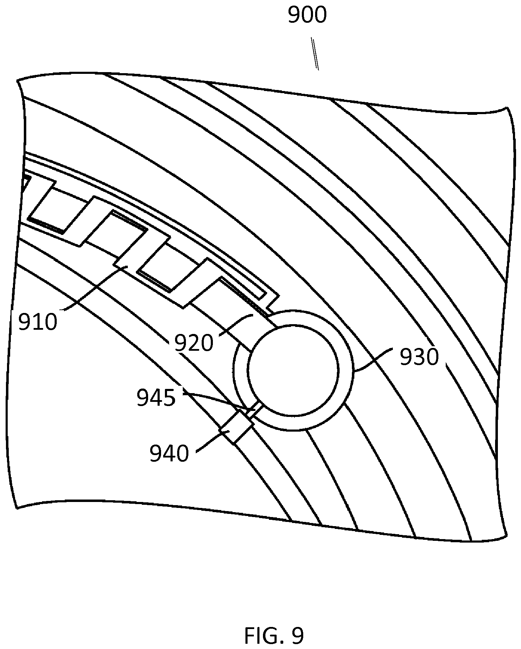

Referring now to Fig. 9, a top view section of an exemplary Microfluidic Analytical System component 900 with a waste or fluid retention vessel 930 is depicted. In the exemplary embodiments, electrode 910 for measuring the flow rate of fluid in the system is an end electrode of many others (not depicted in Fig. 9). Fluid flow through the microchannel 920 and continue to a fluid retention vessel 930. The fluid retention vessel is used, for example, for higher volume of fluid analysis therein. In some embodiments, a pore 940 include a pore control element 945 for connecting the fluid retention vessel 930, which be also be used as a waste storage element, 930 to regions located external to the insert. In addition, in some embodiments the pore control element 945 connection be useful for equalizing gas pressure as the microfluidic components fill with fluid. In other embodiments, the pore 940 and pore control element 945 be useful for emitting fluid from the ophthalmic device. The pore 940 also is useful for connecting an end of the Micro fluidic Analytical System to its external region in an eye environment, which can allow for continuous monitoring without the removal of the ophthalmic device. In other embodiments, the pore 940 and pore control element 945 be useful for flow control through the Micro fluidic Analytical System in a storage location, such as the fluid retention vessel 930. For example, while in storage, the Microfluidic Analytical System may be cleansed or refreshed by the flowing of solutions through the system and, in some embodiments, subjected to calibration protocols. Control of these functions is performed by the integrated circuit components within the lens which also is in communication with external controlling systems.

### Energized Ophthalmic Devices with Lab on a Chip Components

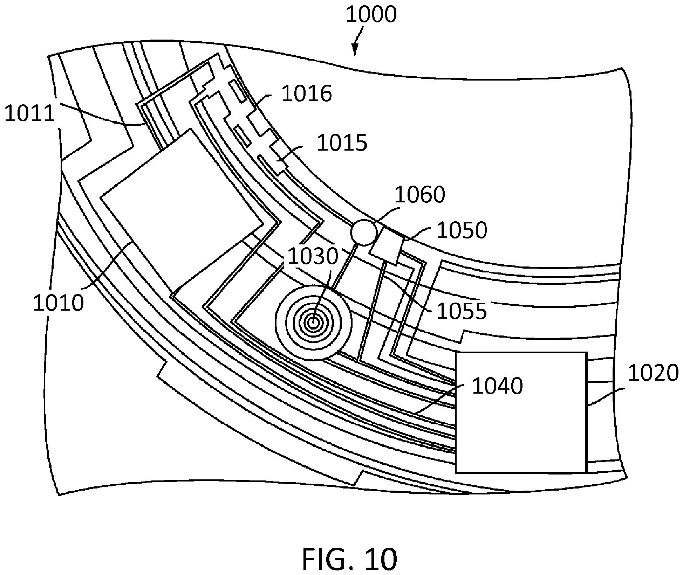

Referring now to Fig. 10, a top view section of an exemplary pumping mechanism 1000 for a Micro fluidic Analytical System using lab on a chip component 1010 is depicted. A lab on a chip component 1010 share many aspects with the embodiment of the Microfluidic Analytical System that has been previously discussed. Similarly, however, in some embodiments small droplets be moved around within the lab on a chip 1010 not through the action of a pump 1060 but by control of the droplets with EWOD components. Droplets are combined in elements of the lab on a chip component 1010 to perform chemical processing. Numerous analysis techniques may be performed, for example, in some embodiments the analysis of glucose as an analyte is performed. The technique for this analysis include, for example, an electrochemical or photometric technique as described or other techniques that relate to the mixing of chemical substances that be initially stored in the lab on a chip component 1010. Quantum-dot based spectroscopy techniques are performed in or through the lab on a chip component. In some examples, QD based dyes be located in a droplet that is mixed with a sample obtained from the environment of the biomedical device. The resulting interaction with a targeted analyte yields spectroscopic signals that be used to calculate a concentration of the analyte for example.

Various components such as energization elements (not shown), interconnects 1040, and sealing aspects previously described take place in the annular Media Insert piece of the present example. Further, an electronic circuit 1020 capable of controlling various components including a lab on a chip component 1010 is implemented. A pore 1050 and a pore control system 1055 control the sampling of fluid samples from the ophthalmic device environment. A pump actuator 1030 actuates a pump 1060 which may be mechanical in nature such as a membrane based pump. Droplets of a fluid sample are pumped into micro-channel 1015 for metering of the volume and sample flow rate through the use of electrodes such as electrode 1016 as described in the present disclosure. The droplets are provided to the lab on a chip component 1010 through a channel 1011 where it is further processed. The lab on a chip component 1010 may use the pumped action on the sample to control flow within itself, or in other embodiments, it control the flow rate of the sample provided to it on its own.

In additional embodiments, the lab on a chip component 1010 is able to sense fluid in its environment without the need of an external pumping system. However, a pore 1050 still is useful to provide control over flow of external fluid into the environment of the lab on a chip component. Thereafter the lab on a chip component 1010 may sample the introduced sample on its own, for example, by the control through electrowetting on dielectric or electrophoresis features that can attract and move fluid samples.

The lab on a chip component 1010 may comprise a design that be consistent with the present description including, for example, very thin lab on chip flexible components to allow for the deformation into a shape consistent with the three dimensional shape of an ocular surface. In some embodiments, the shape and thickness of the lab on a chip component 1010 allow it to be included in a planar form within the ophthalmic insert device.

### Biomedical Devices with Quantum-Dot Spectrometers

Fig. 11A illustrates an exemplary QD Spectrometer system in a biomedical device 1100. The illustration in Fig. 11A may utilize a microfluidic system as illustrated in Fig. 10, or alternatively, may utilize a more passive approach to collecting samples wherein a sample fluid passively enters a channel 1102. The channel 1102 is internal to the biomedical device in some examples and in other examples, as illustrated; the biomedical device surrounds an external region with a reentrant cavity. In some examples where the biomedical device creates a channel of fluid external to itself, the device also contain a pore 1160 to emit reagents or dyes to interact with the external fluid in the channel region. In a non-limiting sense, the passive sampling may be understood with reference to an example where the biomedical device is a swallowable pill. The pill contains regions that emit medicament 1150 as well as regions that analyze surrounding fluid such as gastric fluid for the presence of an analyte, where the analyte be the medicament for example. The pill contains controller 1170 regions proximate to the medicament where control of the release of the medicament is made by portions of the biomedical pill device. An analysis region comprises a reentrant channel within the biomedical pill device that allows external fluid to passively flow in and out of the channel. When an analyte, for example in gastric fluid, diffuses or flows into the channel it becomes located between the analysis region as depicted in Fig. 11A.

Referring now to Fig. 11B, once an analyte diffuses or otherwise enters the Quantum-dot spectrometer channel which shall be referred to as the channel 1102, a sample 1130 pass in the emission portion of a Quantum-dot (QD) emitter 1110. The QD emitters 1110 receive information from a QD emitter controller 1112 instructing the QD emitters 1110 to emit an output spectrum of light across the channel 1102.

In some examples, the QD emitter acts based on emission properties of the quantum-dots. In other examples, the QD emitter act based on the absorption properties of the quantum-dots. In the examples utilizing the emission properties of the quantum-dots, these emissions be photostimulated or electrically stimulated. In some examples of photostimulation; energetic light in the violet to ultraviolet be emitted by a light source and absorbed in the quantum-dots. The excitation in the QD relaxes by emitting photons of characteristic energies in a narrow band. As mentioned previously, the QDs are engineered for the emission to occur at selected frequencies of interest. In a similar set of examples, QDs be formed into the layered sandwiched mentioned previously between electrically active layers that donate electrons and holes into the QDs. These excitations similarly emit characteristic photons of selected frequency. The QD emitter 1110 may be formed by inclusion of nanoscopic crystals, that function as the quantum-dots, where the crystals be controlled in their growth and material that are used to form them before they are included upon the emitter element.

In an alternative set of examples, where the QDs act in an absorption mode a combination of a set of filters be used to determine a spectral response in a region. This mechanism is described in a prior section in reference to Fig. 2. Combinations of QD absorption elements are used in analysis to select regions of the spectrum for analysis.

In either of these types of emission examples, a spectrum of light frequencies is emitted by QD emitter 1110 and pass thru the sample 1130. The sample 1130 absorbs light from some of the emitted frequencies if a chemical constituent within the sample is capable of absorbing these frequencies. The remaining frequencies that are not absorbed continue on to the detector element, where QD receivers 1120 absorb the photons and convert them to electrical signals. These electrical signals are converted to digital information by a QD detector sensor 1122. In some examples the sensor 1122 is connected to each of the QD receivers 1120, or in other examples the electrical signals be routed to centralized electrical circuits for the sensing. The digital data is used in analyzing the sample 1130 based on pre-determined values for QD wavelength absorbance values.

In Fig. 11C, the QD system is depicted in a manner where the sample is passed in front of spectral analysis elements that are spatially located. This may be accomplished for example in the manners described for the microfluidic progression. In other examples, the sample 1130 contain analytes that diffuse inside an region of a biomedical device that encloses external fluid with material of the biomedical device to form a pore or cavity into which the sample passively flow or diffuse to an analytical region that passes light from emitters within the biomedical device, outside the biomedical device, and again to detectors within the biomedical device. Figs. 11B and 11C depict such movement as the difference between the locations of the sample 1130 which has moved along the analysis region to the new location 1131. In other examples the QDs are consolidated to act in a single multidot location where the excitation means and the sensing means are consolidated into single elements for each function. Some biomedical devices such as ophthalmic devices may have space limitations for a spectrometer comprising more than a hundred quantum-dot devices, but other biomedical devices may have hundreds of quantum-dot devices which allow for a full spectrographic characterization of analyte containing mixtures.

The QD analytical system may also function with microfluidic devices to react samples containing analytes with reagents containing dyes. The dye molecules react with specific analytes. As mentioned previously, an example of such a binding is the FRET indicators. The dye molecules have absorption bands in the ultraviolet and visible spectrum that are significantly strong, which also be referred to as having high extinction coefficients. Therefore, small amounts of a particular analyte be selectively bound to molecules that absorb significantly at a spectral frequency, which be focused on by the QD analytical system. The enhanced signal of the dye complex allow for more precise quantification of analyte concentration.

In some examples, a microfluidic processing system mixes an analyte sample with a reagent comprising a dye that will bind to a target analyte. The microfluidic processing system mixes the two samples together for a period that would ensure sufficient complexing between the dye and the analyte. Thereafter, in some examples, the microfluidic processing system moves the mixed liquid sample to a location containing a surface that binds to any uncomplexed dye molecules. When the microfluidic system then further moves the sample mixture into an analysis region, the remaining dye molecules will be correlatable to the concentration of the analyte in the sample. The mixture may be moved in front of either quantum-dot emission light sources or quantum-dot absorption filters in the manners described.

A type of fluorescent dye may be formed by complexing quantum-dots with quenching molecules. A reagent mixture of quantum-dots with complexed quenching molecules is introduced into a sample containing analytes, for example in a microfluidic cell, within a biomedical device. The quenching molecules contain regions that bind to analytes selectively and in so doing separate the quenching molecule from the quantum-dot. The uncomplexed quantum-dot may now fluoresce in the presence of excitation radiation. In some examples, combinations of quantum-dot filters are used to create an ability to detect the presence of enhanced emission at wavelengths characteristic of the uncomplexed quantum-dot. In other examples, other manners of detecting the enhanced emission of the uncomplexed quantum-dots are utilized. A solution of complexed quantum-dots be stored within a microfluidic processing cell of a biomedical device and be used to detect the presence of analytes from a user in samples that are introduced into the biomedical device.

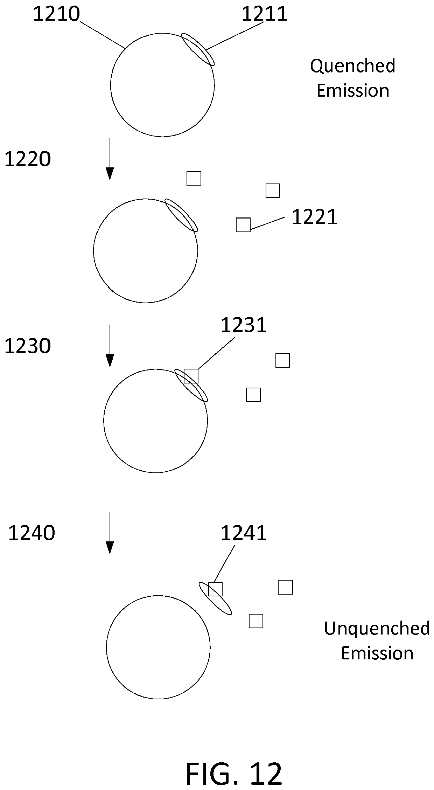

Referring to Fig. 12, an exemplary illustration of the concept of complexed quantum-dots acting as a dye is illustrated. A quantum-dot 1210 comprise an exemplary material such as indium phosphide / zinc sulfide, copper indium sulfide / zinc sulfide, cadmium selenide, cadmium sulfide, lead sulfide, lead selenide, indium arsenide, and indium phosphide as examples. Other examples comprise nanoparticles of silicon and carbon. Any material that may form a strained band structure of the type characteristic of a quantum-dot may be used in some examples. The quantum-dot core may be surrounded by a core shell coating that provides interface from the quantum-dot to its outside environment. For some examples, a biocompatible lipid coating which allow for the binding of quenching molecules to the surface of the dot also be provided. The quenching molecules 1211 are bound to the quantum-dot surface and act to facilitate electronic energy transfer from the quantum-dot which results in a de-excitation of the quantum-dot energy without fluorescent emission. A solution of the quantum-dots is mixed 1220 with a sample containing analytes 1221. During the mixing, analytes complex 1230 with the quenching molecules forming an analyte/quenching molecule complex 1231. The complexing of the analyte with the quenching molecule decouple 1240 the quenching molecule from the quantum-dot, resulting in a free analyte/quenching molecule 1241 and an uncomplexed quantum-dot. Now, the quantum-dot be excited by photons at energy distinct from the inherent fluorescent energy of the quantum-dot and the unquenched quantum-dot will now fluoresce. The concentration of the analyte in the sample is a function of the fluorescence signal emanating from the unquenched quantum-dot. The microfluidic analysis system includes a light source, which be a quantum-dot light emitting diode for example or other light sources with energy distinct from the fluorescence signal. A detector is configured to detect all light that traverses a spectral analysis region. Alternatively, quantum-dot absorbance filters or other light filters may be used to selectively pass the energy band of the quantum-dot fluorescence signal.

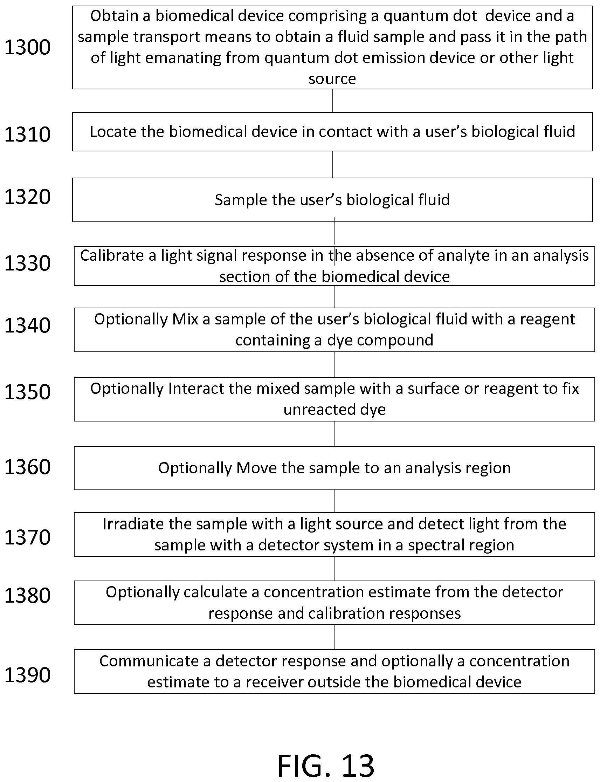

Referring to Fig. 13, an illustration of a flow diagram for analyte analysis in quantum-dot configured biomedical devices is provided. At step 1300, a user obtains a biomedical device comprising: a quantum-dot device or reagent, and a sample transport mean. The biomedical device is capable of obtaining a fluid sample from the user and passing it into the path of light emanating from a quantum-dot emission device or other light source. The light source include a quantum-dot light emitting diode or a set of quenched quantum-dot filters configured to isolate selected spectral regions for analysis. Other light sources such as light emitting diodes and lasers may also be used. In some examples, the biomedical device is obtained by an intermediary for the purposes of use by an end user. At step 1310, the biomedical device is located in contact with a user's biological fluid. The location may include regions proximate to fluid emanations from a user such as, in non-limiting examples, tear fluid, blood, saliva, and waste products. Or, the location may include subcutaneous locations and locations within or in contact with the user's body cavity and venous system. At step 1320, the biomedical device is used to sample the user's biological fluid. At step 1330, the biomedical device engages a calibration protocol either in an autonomous fashion or under direction of an external device or communication signal. The calibration test the biomedical device's sample analysis section in the absence of an analyte to allow for a reference control signal that be used in calculations related to an ultimate sample analysis signal. At step 1340, in some examples, an aliquot of the sample of user's biological fluid be mixed with a reagent comprising a dye compound. The mixing occurs by passive diffusion based interaction, or alternatively, be actively controlled such as with a microfluidic processing system as has been described herein. The dye compound is an organic dye or, in some examples, a quantum-dot based dye. The dye changes a spectral characteristic, such as fluorescence emission or spectral absorbance, when it binds with an analyte in the sample. At step 1350, in some examples, the mixture subsequently is processed to remove unreacted dye, particularly in examples where the binding of the dye to the analyte does not change a spectral characteristic of the dye. The sample is mixed with a reagent that renders unbound dye inert or alternatively the sample be passed in contact with a surface that bind and immobilize or separate out unbound dye. At step 1360, the sample mixture is moved from a reaction region to an analysis region in some examples. In other examples, the same location where reactions occur be used to perform a spectral analysis. At step 1370, the sample is irradiated with a light source. The light source may be from numerous example types including sources comprising quantum-dots as have been described. The irradiation proceed through the sample and light that emerges from the sample be detected in a spectral region by a detector system in the biomedical device. The detected emanation signal be converted into an electrical signal and also be converted into a digital data value that also be conveyed as an electrical signal stream. At 1380, in some examples, the biomedical device include on board processing devices and software algorithms that allow for a calculation of an estimate of a concentration of the analyte in the sample. In other examples, the raw data signals, detector (calibration) signal without analyte, and detector signal with analyte be transmitted without further signal processing in the biomedical device. At step 1390 the raw data signals are communicated, for example, via wireless communication, to an external transceiver. In some examples, at step 1390, a calculated estimate of the concentration of an analyte also is communicated. In still further examples, there are numerous other sensor data that are transmitted in addition to the analysis system data, which may include in a non-limiting perspective sensor measurements of the temperature sensed in a region of the biomedical device.

The QD spectrometer systems may be utilized in several different biomedical devices including: ophthalmic devices, biomedical pills, hygiene products, patches, and other similar biomedical products that are on or in proximity to the body in such a manner to detect and analyze analytes. The information obtained from the QD spectrometer system may be utilized for biometric analysis such as real-time readings of glucose for diabetics, as a non-limiting example. The information obtained may be communicated to a tertiary device, such as a smart phone, as disclosed in relationship to Fig. 4.

### Methods For Monitoring Bioanalytes

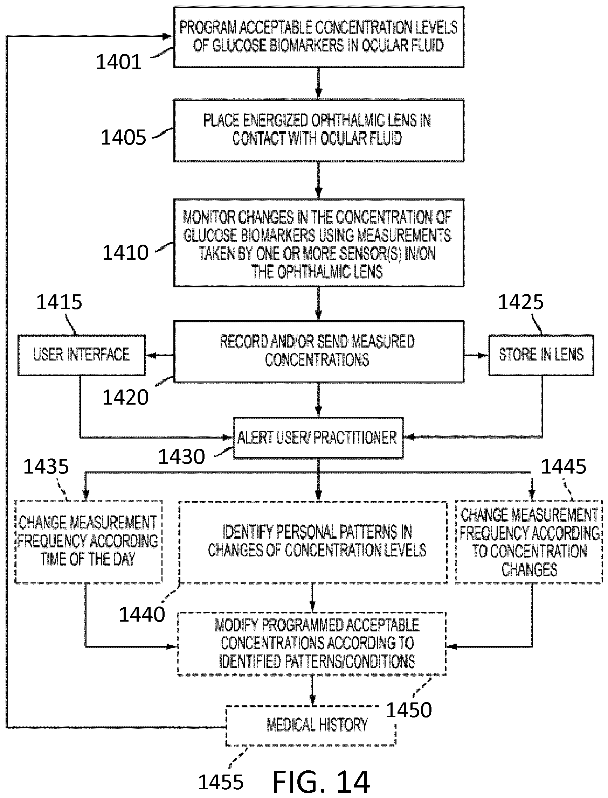

Referring now to Fig. 14, exemplary method steps that be used to monitor analyte levels of a user wearing the ophthalmic lens according to aspects of the present disclosure are illustrated. At step 1401, thresholds values are programmed into a software program. According to aspects of the present invention, threshold values include, for example, acceptable levels for the concentration of glucose biomarkers in ocular fluid. The use of other biomarkers used to monitor different conditions such as depression, high blood pressure, and the like, are also within the inventive scope of aspects of the present disclosure. In addition, the preprogrammed levels may be different depending on whether the ocular fluid sample target is, for example, tear fluid or an interstitial fluid. The program is stored and executed using one or both a processor forming part of the Media Insert of the ophthalmic device and an exterior device in communication with the processor of the Media Insert. An exterior device includes a smart phone device, a PC, a specialized biomedical device user interface, and the like; and be configured to include executable code useful to monitor properties of ocular fluid samples. Ocular fluid properties may be measured by one or more sensors contained in the ophthalmic device. Sensors may include electrochemical sensors and/or photometric sensors. In an exemplary embodiment, the sensor analysis step relate to a photometric sensing of glucose concentration based on a QD spectrometry. In another example, the sensor detects the presence of reaction products from a glucose oxidase interaction with portions of the analyte sensor and the fluid sample.

At step 1405, the ophthalmic device including a microfluidic system be placed in contact with a portion of the anterior ocular surface of the eye and worn by a user. In some embodiments, the ophthalmic device is in a form of an energized contact lens and the step be achieved when the contact lens is placed on the eye surface. In other embodiments, the biomedical device be, for example, in the form of an intraocular lens, punctalplug, biomedical pill, or any other similar biomedical device, and still include aspects of the QD spectrometer system described in the present disclosure. Although the ophthalmic device is described throughout the specification in singular form, it will be understood by one skilled in the art that two ophthalmic devices (e.g. contact lenses), one placed on each eye, may function together to provide functionality aspects of the present disclosure.

At step 1410, concentration changes of biomarkers be monitored using the one or more sensors. The monitoring of the biomarkers occurs at a predetermined frequency/bandwidth or upon demand through a user interface and/or an activation sensor in the ophthalmic device. Biomarkers can include those correlated to glucose levels, depression, blood pressure and the like. At step 1420, the processor of the ophthalmic device can record the measured property/condition from a sample of ocular fluid. In some embodiments, the processor of the ophthalmic device stores it and/or sends it to one or more device(s) in communication with the ophthalmic device. At step 1415, the value recorded can be stored and analyzed in the user interface in communication with the ophthalmic lens, and/or, at step 1425, the analysis and recording can take place in the ophthalmic device.

At step 1430, one or both the ophthalmic device and the user interface alert the user, and/or a practitioner, of the measured concentration. The alert is programmed to occur when the levels measured are outside the predetermined threshold values programmed, received and/or calculated by the ophthalmic device. In addition in some embodiments, the data and alerts are analyzed to perform one or more steps of: a) change measurement frequency according to the time of the day, b) identify personal patters in the changes of concentration levels measures, and c) change the measurement frequency according to the changes in concentrations measured. At step 1435, the time of the day change the frequency of measurements. For example, if the ophthalmic device is one that would remain in the eye during sleep, the number of measurements during 10 pm and 6am can decrease or stop. Similarly, during lunch and dinner times the frequency increase to detect changes due to the food consumption of the user. At step 1440, patterns in changes of the concentration levels are identified by the system. Using the identified patterns, the system alert the user of causes and/or, at step 1445, change the frequency according to the identified changes so that the system is more alert during critical identified conditions. Critical conditions can include events that would trigger a significant increase or decrease in glucose levels. Events can include, for example, holiday dates, exercise, location, time of the day, consumption of medicaments and the like.

In some embodiments, at step 1450, the originally programmed values are customized, periodically or in real time, according to identified patterns/conditions. This ability allows the system to increase its effectiveness by eliminating false alarms and increasing sensitivity at a critical condition. Effectiveness can promote user participation with the system thereby maximizing the benefits of the ophthalmic device and thereby providing a safe monitoring system. At step 1455, data relating to the user including, for example, the identified patterns, measurements, and/or preferences may become part of the medical history of the user. Medical history is stored securely by encrypting the data and/or restricting its access.

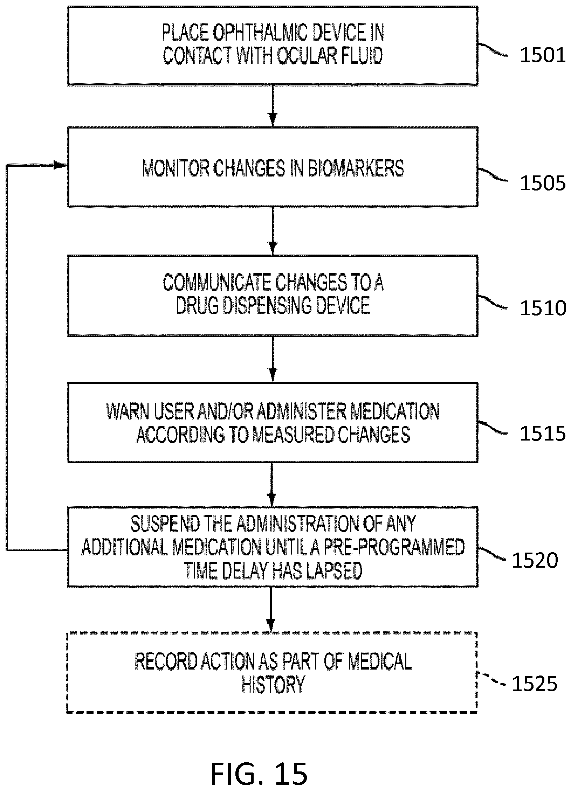

Referring now to Fig. 15, exemplary method steps that may be used to treat the glucose levels of a user wearing the ophthalmic lens according to aspects of the present disclosure are illustrated. At step 1501, an ophthalmic device including a QD spectrometer analytical system is placed in contact with ocular fluid. In some embodiments, the ophthalmic device is in a form of an energized contact lens and the step be achieved when the contact lens is placed on the eye surface. In other embodiments, the ophthalmic device is, for example, in the form of an intraocular lens or a punctal plug, and still includes aspects of the QD spectrometer system described in the present disclosure.

At step 1505, changes in biomarkers in the ocular fluid can be monitored. Methods of monitoring the biomarker changes include, for example, steps illustrated in Fig. 14. At step 1510, measured changes can be communicated in real time to a medicament-dispensing device in direct or indirect communication with the ophthalmic device. Although the changes in concentration of the monitored biomarkers in ocular fluid may include a time delay in relation to the concentration changes in the bloodstream of the user, upon detection, at step 1515 the medicament-dispensing device administer a medicament capable of lowering or raising concentrations to a normal level. For example, glucose levels be monitored and treated when they are outside a normal level. Continuous monitoring prevent uncontrolled blood sugar levels which may damage the vessels that supply blood to important organs, like the heart, kidneys, eyes, and nerves. Because an individual whose glucose levels reach a level that exposes him/her to the risks may feel fine, aspects of the present disclosure help take action upon early detection of the condition. Early detection not only bring back levels to a normal condition and/or make the user aware, but additionally prevent the more dramatic and permanent consequences including, for example, a heart attack or stroke, kidney failure, and blindness which have been known to occur when abnormal glucose levels are left untreated.

In addition, in some embodiments the medicament administering device sends an alert to the user through its interface or using component of the ophthalmic device. For example, in some ophthalmic device embodiments the Media Insert include a light projection system, such as one or more LEDs, capable of sending a signal to the user.

Subsequently at step 1520, any further drug administering can be suspended to prevent overdosing of the system due to the time delay of the effect of the drug and the effect to be reflected in the tear fluid. For example, the medicament requires 10-30 minutes to counteract the abnormal level, and upon its effect, take another 20 minutes to equalize concentrations in tear fluid. Consequently, programmed algorithms capable of correlating the condition, time delay, and appropriate subsequent dosing of medicaments can be programmed in the system to function safely. At step 1525, data relating to one or both the measured conditions and the medicament administration to the user may be stored and used as part of a treatment and/or medical history of the user.

Specific examples have been described to illustrate sample embodiments for the methods and devices related to inclusion of quantum-dots for spectroscopic analysis in biomedical devices. These examples are for said illustration and are not intended to limit the scope of the claims in any manner. Accordingly, the description is intended to embrace all examples that may be apparent to those skilled in the art.

## Claims

1. A biomedical device for analyzing an absorbance of an analyte in a sample (1130) of bodily fluid, comprising:
an energization element (110) including a first and second current collector, a cathode, an anode, and an electrolyte;
a quantum-dot light emitter(1110) configured to emit photons of characteristic energies in a narrow wavelength band which pass through the sample, wherein the quantum-dot emitter is powered by the energization element;
a photodetector (1120) configured to receive transmitted photons which are not absorbed by the sample, and
an integrated circuit controller (1112) within the biomedical device wherein the integrated circuit controller is capable of directing a functionality of the quantum-dot emitter and photodetector,
wherein the absorbance of the analyte in the sample of bodily fluid is analyzed based on the intensity of photons received by the photodetector.

2. The biomedical device of claim 1, comprising:
a quantum dot spectrometer including the quantum-dot light emitter, the photodetector, and a means of communicating information from the quantum-dot spectrometer to a user; and
an insert device (100), wherein the insert device contains the energization element and the quantum-dot spectrometer, and wherein the insert device isolates the energization element from a biomedical environment in which the biomedical device operates.

3. The biomedical device of claim 2 further comprising a micro-fluidic pump (760) wherein the micro-fluidic pump functions to bring the sample of bodily fluid towards or away from the quantum-dot spectrometer for analysis.

4. The biomedical device of claim 2 wherein the biomedical device is an ophthalmic device (150).

5. The biomedical device of claim 2 wherein the biomedical device is a contact lens.

6. The biomedical device of claim 2 wherein the biomedical device is an electronic pill.

7. A method of analyzing an analyte in a sample (1130) of bodily fluid using a biomedical device according to claim 1, the method comprising:
emitting photons of characteristic energies in a narrow wavelength band from the quantum-dot light emitter;
receiving transmitted photons which are not absorbed by a sample of analytes into the photodetector; and
analyzing the absorbance of the analyte based on the intensity of photons received.

8. The method of claim 7 further comprising pumping a sample (1130) of analytes into a quantum-dot spectrometer channel (1102) before analyzing the analytes.

9. The method of claim 7 wherein the biomedical device is a contact lens.

10. The method of claim 7 wherein the biomedical device is an electronic pill.

11. The biomedical device of claim 1, further comprising:
an external encapsulation boundary, wherein at least a portion of the boundary forms a reentrant cavity, wherein a sidewall of the cavity allows light to pass through in a selected spectral band, wherein the quantum-dot light emitter (1110) is installed to emit light through one side of the sidewall of the cavity through an intervening space of the cavity and through a distal side of the sidewall of the cavity, and the photodetector (1120) is installed on the distal side of the cavity within the external encapsulation boundary;
a radio frequency transceiver (445); and
an analog-to-digital converter, wherein a signal from the photodetector is converted to a digital data value that is transmitted outside the biomedical device by the radio frequency transceiver.

12. The biomedical device of claim 11 wherein the device is a contact lens.

13. The biomedical device of claim 11 wherein the device is an electronic pill.

14. The biomedical device of claim 13 wherein the electronic pill comprises a release mechanism (1170) controllable to release medicament (1150) based on the signal received at the photodetector.

15. The biomedical device of claim 14 wherein the signal received at the photodetector is converted to a digital signal and communicated to an external receiver, wherein at the external receiver an algorithm calculates a concentration of the analyte and determines a time value for release of medicament (1150).

16. The biomedical device of claim 13 wherein the electronic pill comprises a release mechanism (1160) controllable to release a quantum-dot dye into the cavity, wherein the dye reacts with analyte molecules and allows the quantum light emitter (1110) to excite the quantum-dot dye to emit light.

17. The biomedical device of claim 1, further comprising:
an external encapsulation boundary, wherein at least a portion of the boundary comprises an electrically controlled pore (1050) operative to allow the fluid sample to pass into the biomedical device from an external region;
a microfluidic processing chip (1010) operative to mix the fluid sample with a reagent comprising an analyte specific dye, wherein the quantum-dot light emitter (1110) is installed to emit light through a portion of the micro fluidic processing chip and the photodetector (1120) is installed on a distal side of the micro fluidic processing chip from the quantum-dot light emitter, wherein light emitted by the quantum-dot light emitter proceeds through a top surface of the micro fluidic processing chip, through a sample analysis region of the micro fluidic processing chip, through a bottom surface of the micro fluidic processing chip and into the photodetector;
a radio frequency transceiver (445); and
an analog-to-digital converter, wherein a signal from the photodetector is converted to a digital data value that is transmitted outside the biomedical device by the radio frequency transceiver.

18. The biomedical device of claim 17 wherein the device is a contact lens.

19. The biomedical device of claim 17 wherein the device is an electronic pill.

20. The biomedical device of claim 19 wherein the electronic pill comprises a release mechanism controllable to release medicament (1150) based on the signal received at the photodetector.

21. The biomedical device of claim 19 wherein the electronic pill comprises a release mechanism (1160) controllable to release a quantum-dot dye into the microfluidic processing chip, wherein the dye reacts with analyte molecules and allows the quantum light emitter (1110) to excite the quantum-dot dye to emit light whose intensity is correlated to a concentration of the analyte molecules.

## Patentansprüche

1. Biomedizinische Vorrichtung zur Analyse eines Absorptionsvermögens eines Analyten in einer Probe (1130) von Körperflüssigkeit, umfassend:
ein Erregungselement (110) mit einem ersten und einem zweiten Stromabnehmer, einer Kathode, einer Anode und einem Elektrolyten;
einen Quantenpunkt-Lichtsender (1110), der zum Aussenden von Photonen charakteristischer Energien in einem schmalen Wellenlängenband, die die Probe durchlaufen, ausgelegt ist, wobei der Quantenpunkt-Sender vom Erregungselement angetrieben wird;
einen Photodetektor (1120), der dazu ausgelegt ist, übertragene Photonen zu empfangen, die von der Probe nicht absorbiert werden, und
eine integrierte Schaltungssteuerung (1112) innerhalb der biomedizinischen Vorrichtung, wobei die integrierte Schaltungssteuerung dazu befähigt ist, eine Funktionalität des Quantenpunkt-Senders und Photodetektors zu lenken,
wobei das Absorptionsvermögen des Analyten in der Körperflüssigkeitsprobe auf Basis der Intensität der vom Photodetektor empfangenen Photonen analysiert wird.

2. Biomedizinische Vorrichtung nach Anspruch 1, umfassend:
ein Quantenpunkt-Spektrometer, das den Quantenpunkt-Lichtsender, den Photodetektor und ein Mittel zur Übermittlung von Informationen vom Quantenpunkt-Spektrometer an einen Benutzer aufweist; und
eine Einsatzvorrichtung (100), wobei die Einsatzvorrichtung das Erregungselement und das Quantenpunkt-Spektrometer enthält, und wobei die Einsatzvorrichtung das Erregungselement von einer biomedizinischen Umgebung, in der die biomedizinische Vorrichtung arbeitet, isoliert.

3. Biomedizinische Vorrichtung nach Anspruch 2, ferner umfassend eine Mikrofluidikpumpe (760), wobei die Mikrofluidikpumpe dazu dient, die Körperflüssigkeitsprobe zur Analyse zum Quantenpunkt-Spektrometer hin oder von diesem weg zu führen.

4. Biomedizinische Vorrichtung nach Anspruch 2, wobei die biomedizinische Vorrichtung eine ophthalmische Vorrichtung (150) ist.

5. Biomedizinische Vorrichtung nach Anspruch 2, wobei die biomedizinische Vorrichtung eine Kontaktlinse ist.

6. Biomedizinische Vorrichtung nach Anspruch 2, wobei die biomedizinische Vorrichtung eine elektronische Pille ist.

7. Verfahren zur Analyse eines Analyten in einer Probe (1130) von Körperflüssigkeit unter Verwendung einer biomedizinischen Vorrichtung nach Anspruch 1, wobei das Verfahren das Folgende umfasst:
Senden von Photonen charakteristischer Energien in einem schmalen Wellenlängenband vom Quantenpunkt-Lichtsender;
Empfangen übertragener Photonen, die von einer Analytenprobe nicht absorbiert wurden, in den Photodetektor; und
Analyse des Absorptionsvermögens des Analyten auf Basis der Intensität der empfangenen Photonen.

8. Verfahren nach Anspruch 7, ferner umfassend das Pumpen einer Probe (1130) von Analyten in einen Kanal (1102) des Quantenpunkt-Spektrometers vor der Analyse der Analyten.

9. Verfahren nach Anspruch 7, wobei die biomedizinische Vorrichtung eine Kontaktlinse ist.

10. Verfahren nach Anspruch 7, wobei die biomedizinische Vorrichtung eine elektronische Pille ist.

11. Biomedizinische Vorrichtung nach Anspruch 1, ferner umfassend:
eine externe Verkapselungsgrenze, wobei zumindest ein Teil der Grenze eine Wiedereintrittshöhle bildet, wobei eine Seitenwand der Höhle das Durchdringen von Licht in einem ausgewählten Spektralband gestattet, wobei der Quantenpunkt-Lichtsender (1110) zum Senden von Licht durch eine Seite der Seitenwand der Höhle durch einen Zwischenraum der Höhle und durch eine distale Seite der Seitenwand der Höhle angebracht ist und der Photodetektor (1120) auf der distalen Seite der Höhle innerhalb der externen Verkapselungsgrenze angebracht ist;
einen Radiofrequenz-Sende-Empfänger (445); und
einen Analog-Digital-Wandler, wobei ein Signal vom Photodetektor in einen digitalen Datenwert umgewandelt wird, der außerhalb der biomedizinischen Vorrichtung vom Radiofrequenz-Sende-Empfänger übertragen wird.

12. Biomedizinische Vorrichtung nach Anspruch 11, wobei die Vorrichtung eine Kontaktlinse ist.

13. Biomedizinische Vorrichtung nach Anspruch 11, wobei die Vorrichtung eine elektronische Pille ist.

14. Biomedizinische Vorrichtung nach Anspruch 13, wobei die elektronische Pille einen Freisetzungsmechanismus (1170) umfasst, der auf Basis des am Photodetektor empfangenen Signals zur Freisetzung eines Medikaments (1150) steuerbar ist.

15. Biomedizinische Vorrichtung nach Anspruch 14, wobei das am Photodetektor empfangene Signal in ein digitales Signal umgewandelt wird und einem externen Empfänger übermittelt wird, wobei am externen Empfänger ein Algorithmus eine Konzentration des Analyten berechnet und einen Zeitwert zur Freisetzung von Medikament (1150) bestimmt.

16. Biomedizinische Vorrichtung nach Anspruch 13, wobei die elektronische Pille einen Freisetzungsmechanismus (1160) umfasst, der zur Freisetzung eines Quantenpunkt-Farbstoffs in die Höhle steuerbar ist, wobei der Farbstoff mit Analytenmolekülen reagiert und es dem Quantenpunkt-Lichtsender (1110) erlaubt, den Quantenpunkt-Farbstoff zum Senden von Licht zu erregen.

17. Biomedizinische Vorrichtung nach Anspruch 1, ferner umfassend:
eine externe Verkapselungsgrenze, wobei zumindest ein Teil der Grenze eine elektrisch gesteuerte Pore (1050) umfasst, die dazu dient, das Durchlaufen der Flüssigkeitsprobe in die biomedizinische Vorrichtung von einer externen Region zu erlauben;
einen mikrofluidischen Prozessor-Chip (1010), der dazu dient, die Flüssigkeitsprobe mit einem Reagenz zu mischen, das einen analyten-spezifischen Farbstoff umfasst, wobei der Quantenpunkt-Lichtsender (1110) zum Senden von Licht durch einen Teil des mikrofluidischen Prozessor-Chips angebracht ist und der Photodetektor (1120) auf einer distalen Seite des mikrofluidischen Prozessor-Chips vom Quantenpunkt-Lichtsender angebracht ist, wobei das vom Quantenpunkt-Lichtsender gesendete Licht durch eine Oberseite de mikrofluidischen Prozessor-Chips, durch eine Probenanalyseregion des mikrofluidischen Prozessor-Chips, durch eine Unterseite des mikrofluidischen Prozessor-Chips und in den Photodetektor fortschreitet;
einen Radiofrequenz-Sende-Empfänger (445); und
einen Analog-Digital-Wandler, wobei ein Signal vom Photodetektor in einen digitalen Datenwert umgewandelt wird, der außerhalb der biomedizinischen Vorrichtung vom Radiofrequenz-Sende-Empfänger übertragen wird.

18. Biomedizinische Vorrichtung nach Anspruch 17, wobei die Vorrichtung eine Kontaktlinse ist.

19. Biomedizinische Vorrichtung nach Anspruch 17, wobei die Vorrichtung eine elektronische Pille ist.

20. Biomedizinische Vorrichtung nach Anspruch 19, wobei die elektronische Pille einen Freisetzungsmechanismus umfasst, der auf Basis des am Photodetektor empfangenen Signals zur Freisetzung eines Medikaments (1150) steuerbar ist.

21. Biomedizinische Vorrichtung nach Anspruch 19, wobei die elektronische Pille einen Freisetzungsmechanismus (1160) umfasst, der zur Freisetzung eines Quantenpunkt-Farbstoffs in den mikrofluidischen Prozessor-Chip steuerbar ist, wobei der Farbstoff mit Analytenmolekülen reagiert und es dem Quantenpunkt-Lichtsender (1110) erlaubt, den Quantenpunkt-Farbstoff zum Senden von Licht zu erregen, dessen Intensität mit einer Konzentration der Analytenmoleküle korreliert wird.

## Revendications

1. Dispositif biomédical pour analyser une absorbance d'un analyte dans un échantillon (1130) d'un fluide corporel, comprenant :
un élément d'excitation (110) comprenant un premier et un second collecteur de courant, une cathode, une anode et un électrolyte ;
un émetteur de lumière à points quantiques (1110) configuré pour émettre des photons d'énergies caractéristiques dans une bande de longueur d'onde étroite qui passent à travers l'échantillon, l'émetteur à points quantiques étant alimenté par l'élément d'excitation ;
un photodétecteur (1120) configuré pour recevoir des photons transmis qui ne sont pas absorbés par l'échantillon, et
un dispositif de commande de circuit intégré (1112) à l'intérieur du dispositif biomédical, le dispositif de commande de circuit intégré étant capable de diriger une fonctionnalité de l'émetteur à points quantiques et du photodétecteur,
l'absorbance de l'analyte dans l'échantillon de fluide corporel étant analysée sur la base de l'intensité des photons reçus par le photodétecteur.

2. Dispositif biomédical selon la revendication 1, comprenant :
un spectromètre à points quantiques comprenant l'émetteur de lumière à points quantiques, le photodétecteur, et un moyen de communication d'informations du spectromètre à points quantiques à un utilisateur ; et
un dispositif d'insertion (100), le dispositif d'insertion contenant l'élément d'excitation et le spectromètre à points quantiques, et le dispositif d'insertion isolant l'élément d'excitation d'un environnement biomédical dans lequel le dispositif biomédical fonctionne.

3. Dispositif biomédical selon la revendication 2 comprenant en outre une pompe micro-fluidique (760), la pompe micro-fluidique fonctionnant pour amener l'échantillon de fluide corporel en direction ou à l'opposé du spectromètre à points quantiques pour analyse.

4. Dispositif biomédical selon la revendication 2, le dispositif biomédical étant un dispositif ophtalmique (150).

5. Dispositif biomédical selon la revendication 2, le dispositif biomédical étant une lentille de contact.

6. Dispositif biomédical selon la revendication 2, le dispositif biomédical étant une pilule électronique.

7. Procédé d'analyse d'un analyte dans un échantillon (1130) d'un fluide corporel à l'aide d'un dispositif biomédical selon la revendication 1, le procédé comprenant :
l'émission de photons d'énergies caractéristiques dans une bande de longueur d'onde étroite à partir de l'émetteur de lumière à points quantiques ;
la réception de photons transmis qui ne sont pas absorbés par un échantillon d'analytes dans le photodétecteur ; et
l'analyse de l'absorbance de l'analyte sur la base de l'intensité des photons reçus.

8. Procédé selon la revendication 7, comprenant en outre le pompage d'un échantillon (1130) d'analytes dans un canal de spectromètre à points quantiques (1102) avant l'analyse des analytes.

9. Procédé selon la revendication 7, le dispositif biomédical étant une lentille de contact.

10. Procédé selon la revendication 7, le dispositif biomédical étant une pilule électronique.

11. Dispositif biomédical selon la revendication 1, comprenant en outre :
une limite d'encapsulation externe, au moins une partie de la limite formant une cavité rentrante, une paroi latérale de la cavité permettant à la lumière de passer à travers dans une bande spectrale sélectionnée, l'émetteur de lumière à points quantiques (1110) étant installé pour émettre de la lumière à travers un côté de la paroi latérale de la cavité à travers un espace intermédiaire de la cavité et à travers un côté distal de la paroi latérale de la cavité, et le photodétecteur (1120) étant installé sur le côté distal de la cavité à l'intérieur de la limite d'encapsulation externe ;
un émetteur-récepteur radiofréquence (445) ; et
un convertisseur analogique-numérique, un signal provenant du photodétecteur étant converti en une valeur de données numériques qui est transmise à l'extérieur du dispositif biomédical par l'émetteur-récepteur radiofréquence.

12. Dispositif biomédical selon la revendication 11, le dispositif étant une lentille de contact.

13. Dispositif biomédical selon la revendication 11, le dispositif étant une pilule électronique.

14. Dispositif biomédical selon la revendication 13, la pilule électronique comprenant un mécanisme de libération (1170) pouvant être commandé pour libérer un médicament (1150) sur la base du signal reçu au niveau du photodétecteur.

15. Dispositif biomédical selon la revendication 14, le signal reçu au niveau du photodétecteur étant converti en un signal numérique et communiqué à un récepteur externe, au niveau du récepteur externe, un algorithme calculant une concentration de l'analyte et déterminant une valeur temporelle pour la libération de médicament (1150).

16. Dispositif biomédical selon la revendication 13, la pilule électronique comprenant un mécanisme de libération (1160) pouvant être commandé pour libérer un colorant à points quantiques dans la cavité, le colorant réagissant avec les molécules d'analyte et permettant à l'émetteur de lumière quantique (1110) d'exciter le colorant à points quantiques pour émettre de la lumière.

17. Dispositif biomédical selon la revendication 1 comprenant en outre :
une limite d'encapsulation externe, au moins une partie de la limite comprenant un pore à commande électrique (1050) fonctionnelle pour permettre à l'échantillon de fluide de passer dans le dispositif biomédical à partir d'une région externe ;
une puce de traitement micro-fluidique (1010) fonctionnelle pour mélanger l'échantillon de fluide avec un réactif comprenant un colorant spécifique d'un analyte, l'émetteur de lumière à points quantiques (1110) étant installé pour émettre de la lumière à travers une partie de la puce de traitement micro-fluidique et du photodétecteur (1120) étant installé sur un côté distal de la puce de traitement micro-fluidique à partir de l'émetteur de lumière à points quantiques, la lumière émise par l'émetteur de lumière à points quantiques passant à travers une surface supérieure de la puce de traitement micro-fluidique, à travers une région d'analyse d'échantillon de la puce de traitement micro-fluidique, à travers une surface inférieure de la puce de traitement micro-fluidique et dans le photodétecteur ;
un émetteur-récepteur radiofréquence (445) ; et
un convertisseur analogique-numérique, un signal provenant du photodétecteur étant converti en une valeur de données numériques qui est transmise à l'extérieur du dispositif biomédical par l'émetteur-récepteur radiofréquence.

18. Dispositif biomédical selon la revendication 17, le dispositif étant une lentille de contact.

19. Dispositif biomédical selon la revendication 17, le dispositif étant une pilule électronique.

20. Dispositif biomédical selon la revendication 19, la pilule électronique comprenant un mécanisme de libération pouvant être commandé pour libérer un médicament (1150) sur la base du signal reçu au niveau du photodétecteur.

21. Dispositif biomédical selon la revendication 19, la pilule électronique comprenant un mécanisme de libération (1160) pouvant être commandé pour libérer un colorant à points quantiques dans la puce de traitement micro-fluidique, le colorant réagissant avec les molécules d'analyte et permettant à l'émetteur de lumière quantique (1110) d'exciter le colorant à points quantiques pour émettre une lumière dont l'intensité est corrélée à une concentration des molécules d'analyte.
